# EUROPÄISCHE PATENTSCHRIFT

(11) **EP 1 317 488 B1**
(45) Veröffentlichungstag und Bekanntmachung des Hinweises auf die Patenterteilung: **27.02.2013**
(21) Anmeldenummer: 01984646.8
(22) Anmeldetag: 07.09.2001
(51) Int. Cl.: C07K 14/525, A61K 47/48, C07K 14/705

(54) **ORTSSPEZIFISCHE, ANTIKÖRPERVERMITTELTE AKTIVIERUNG PROAPOPTOTISCHER ZYTOKINE: AMAIZe (ANTIBODY-MEDIATED APOPTOSIS INDUCING ZYTOKINE)**
SITE-SPECIFIC, ANTIBODY-MEDIATED ACTIVATION OF PROAPOPTOTIC CYTOKINE: AMAICE (ANTIBODY-MEDIATED APOPTOSIS INDUCING CYTOKINE)
ACTIVATION LOCALEMENT SPECIFIQUE, PAR L'INTERMEDIAIRE D'ANTICORPS, D'UNE ZYTOKINE PRO-APOPTOSE : AMAIZE (ANTIBODY-MEDIATED APOPTOSIS INDUCING ZYTOKINE)

(30) Priorität: 15.09.2000 DE 10045591
(43) Veröffentlichungstag der Anmeldung: 11.06.2003
(73) Patentinhaber: BioNTech AG, 55131 Mainz (DE)
(72) Erfinder: PFIZENMAIER, Klaus, 75233 Tiefenbronn (DE); WAJANT, Harald, 70771 Leinfelden-Echterdingen (DE); MOOSMAYER, Dieter, 13359 Berlin (DE); WÜEST, Thomas, CH-8953 Dietikon (CH)
(74) Vertreter: Schnappauf, Georg
(86) Internationale Anmeldenummer: PCT/EP2001/010364
(87) Internationale Veröffentlichungsnummer: WO 2002/022680

(56) Entgegenhaltungen:
- WO-A-00/26244
- WO-A-99/25834
- WO-A-99/43713
- US-A- 5 650 150
- XIANG J: "TARGETING CYTOKINES TO TUMORS TO INDUCE ACTIVE ANTITUMOR IMMUNE RESPONSES BY RECOMBINANT FUSION PROTEINS" HUMAN ANTIBODIES, AMSTERDAM, NL, Bd. 9, 1996, Seiten 23-36, XP000940526 ISSN: 1093-2607
- GILLIES S D ET AL: "Biological activity and in vivo clearance of antitumor antibody/cytokine fusion proteins" BIOCONJUGATE CHEMISTRY, AMERICAN CHEMICAL SOCIETY, WASHINGTON, US, Bd. 4, Nr. 3, Mai 1993 (1993-05), Seiten 230-235, XP002107032 ISSN: 1043-1802
- XIANG J ET AL: "Genetic engineering of a recombinant fusion possessing anti-tumor F(ab@?)2 and tumor necrosis factor" JOURNAL OF BIOTECHNOLOGY, ELSEVIER SCIENCE PUBLISHERS, AMSTERDAM, NL, Bd. 53, Nr. 1, 28. Februar 1997 (1997-02-28), Seiten 3-12, XP004094618 ISSN: 0168-1656
- WILEY S R ET AL: "IDENTIFICATION AND CHARACTERIZATION OF A NEW MEMBER OF THE TNF FAMILY THAT INDUCES APOPTOSIS" IMMUNITY, CELL PRESS, US, Bd. 3, Nr. 6, 1. Dezember 1995 (1995-12-01), Seiten 673-682, XP000672297 ISSN: 1074-7613

## Beschreibung

Die vorliegende Erfindung betrifft Polypeptide, welche als solche biologisch inaktiv oder wenig aktiv sind und erst durch ortsspezifische und antikörpervermittelte Bindung entsprechend aktiviert werden, enthaltend eine Region, welche ein Peptidlinker ist, weiter enthaltend eine Antikörper- bzw. eine davon abstammende Region, die ein spezifisches Molekül auf einer Zelloberfläche selektiv erkennt und weiter enthaltend ein Zytokinanteil, der für sich alleine genommen biologisch inaktiv bzw. nur eingeschränkt aktiv ist. Weiterhin betrifft die vorliegende Erfindung den Polypeptiden zugrundeliegende Nukleinsäuresequenzen, Vektoren, die diese erfindungsgemäßen Nukleinsäuresequenzen enthalten, mit erfindungsgemäßen Nukleinsäuresequenzen oder Vektoren transfizierte Zellen, Verwendungen erfindungsgemäßer Gegenstände zu therapeutischen Zwecken und Zusammensetzungen, enthaltend erfindungsgemäße Gegenstände.

Zytokine, bspw. Mitglieder der TNF-Ligandenfamilie, bspw. TRAIL (TNF Related Apoptosis Inducing Ligand ), auch Apo2L genannt (Wiley et al. (1995), Immunity 6: 673-682, Pitti et al. (1996) J Biol Chem 271: 12687-12689), und bspw. FasL zeigen in in vitro Untersuchungen eine starke apoptotische Wirkung auf viele Tumorzellen tierischen und menschlichen Ursprungs. Im Falle von TRAIL scheint es so zu sein, dass nicht maligne Zellen nicht beeinträchtigt werden. In den untersuchten präklinischen Tiermodellen (Maus, Affe) wurden darüber hinaus keinerlei Anhaltspunkte für eine akute Toxizität oder andere systemische Nebenwirkungen von TRAIL, die als therapiebegrenzend anzusehen wären, festgestellt (Walczak et al. (1999) Nat Med 5:157-163, Ashkenazi et al. (1999) J Clin Invest 104: 155-162). Neuere in vitro Untersuchungen an primären humanen Hepatozyten zeigten allerdings eine starke zytotoxische Wirkung am Beispiel eines rekombinant hergestellten TRAIL-Produktes bzw. von membranständigem TRAIL der natürlich vorkommenden Form dieses Zytokins (Jo et al. (2000) Nat Med 6: 564-567, Ichikawa et al. (2001) Nat Med 7: 954-960). Damit ist eine direkte klinische Anwendung der bisher vorliegenden Zytokine, bspw. rekombinanter TRAIL-Moleküle, welche die Wirkung des membranständigen TRAIL vollständig mimikrieren, ausgeschlossen. Darüber hinaus wurde auch für das verwandte Molekül FasL (Ligand des Fas-Rezeptors (Fas, CD95)), dem Prototyp apoptotischer Zytokine, eine klinische Anwendung a priori aus Sicherheitsgründen unterlassen, da agonistische Antikörper gegen seinen Rezeptor, Fas, in vivo extrem hepatotoxisch sind (Ogasawara et al.(1993) Nature 364: 806-809). Schließlich wurde auch gezeigt, dass FasL in löslicher Form praktisch im Unterschied zu seiner membranständigen Form keine Bioaktivität besitzt (Schneider et al. (1998) J. Exp. Med. 187: 1205-1213).

Damit sind die nach dem Stand der Technik verfügbaren Mitglieder der TNF-Ligandenfamilie entweder auf Grund mangelnder Bioaktivität oder extremer Toxizität zur therapeutischen Anwendung, bspw. zur Behandlung von Tumoren, nicht oder nur sehr begrenzt (z.B. im Falle von TNF unter sog. "isolated limb perfusion" Bedingungen) einsetzbar.

Der vorliegenden Erfindung liegt nunmehr die Aufgabe zugrunde, die Zytokinwirkung gerichtet und gewebs- bzw. zellspezifisch zu entfalten und damit unerwünschte, u.U. systemische Nebenwirkungen auf nicht zum Zielgewebe gehörigen Geweben/Zellen bei einer klinischen Anwendung zu vermeiden oder zumindest stark einzuschränken.

Diese Aufgabe wird erfindungsgemäß durch die Gegenstände der Ansprüche gelöst. Hierbei liegt der vorliegenden Erfindung die Tatsache zugrunde, dass natürlicherweise membranständig vorkommende Zytokine, wenn sie vom Organismus in eine lösliche, der extrazellulären Domäne entsprechende Form proteolytisch prozessiert werden, entweder biologisch gänzlich inaktiv oder nur noch eingeschränkt, z.B auf bestimmten Membranrezeptor-Subtypen, wirksam sind. Dies gilt auch für rekombinant hergestellte Derivate, die der extrazellulären Domäne des jeweiligen Liganden entsprechen. Erfindungsgemäße Erkenntnis ist es, dass ein derart inaktives/eingeschränkt aktives Zytokin durch bspw. Antikörper-vermittelte spezifische Bindung an ein zellmembranständiges Antigen wieder (volle) biologische Wirksamkeit erlangt und zwar gegenüber der Zielzellen selbst wie auch gegenüber benachbart liegenden Zellen, sofern diese jeweils die entsprechenden Zytokinrezeptoren für das eingesetzte Antikörper-Zytokin-Fusionsprotein exprimieren.

Erfindungsgemäß wird ein Polypeptid mit proapoptotischen und immunmodulierenden Eigenschaften bereitgestellt, dadurch gekennzeichnet, dass es (i) einen Abschnitt (1), bestehend aus der Aminosäuresequenz einer extrazellulären Domäne eines Mitglieds der TNF-Familie, (ii) N-terminal von Abschnitt (1) einen Abschnitt (2), welcher ein Peptidlinker ist, und (iii) einen Abschnitt (3), welcher ein an ein zellmembranständiges Antigen bindendes Antikörperfragment ist, enthält, wobei das Polypeptid einen zellmembranständigen Zytokinrezeptor erkennt, und wobei das Antikörperfragment als scFv vorliegt.

Hierbei liegt Abschnitt (3) am N-Terminus eines erfindungsgemäßen Polypeptids, mit zunächst C-terminal nachfolgendem Abschnitt (2) und einem weiter C-terminal angesiedelten Abschnitt (3). Derartige erfindungsgemäße Polypeptide (= erfindungsgemäße Fusionsproteine, = erfindungsgemäße Konstrukte) sind ohne ortsspezifische und/oder selektive Bindung des Abschnitts (3) an das Zielmolekül biologisch inaktiv/eingeschränkt aktiv.

Ganz besonders bevorzugt ist als Abschnitt (1) eine extrazelluläre Domäne, von TRAIL (TNF Related Apoptosis Inducing Ligand, AA 95-281, NCBI Accession No AAC50332 U37518) oder von FasL (AA 139-281, NCBI Accession No. AAC50124 U11821).

Das Wirkprinzip derartiger erfindungsgemäßer Konstrukte, wie beispielweise erfindungsgemäße Konstrukte mit dem Apoptoseinduktor TRAIL oder FasL als Abschnitt (1), ist insbesondere für all diejenigen Mitglieder der TNF-Ligandenfamilie zutreffend, die für bestimmte Rezeptoren ausschließlich oder in besonders gutem Maße als Membranmolekül wirksam sind. Hierzu gehören neben TRAIL auch TNF (in Analogie zu TRAIL den TNF-R2 betreffend) und beispielsweise auch die Immunmodulatoren CD40L und CD30L. Besonders bevorzugt sind daher solche erfindungsgemäßen Polypeptide, die als spezifisches Zielmotekül einen zellmembranständigen Zytokinrezeptor erkennen. Hierzu gehören bspw. auch in einer nicht abschließenden Aufzählung die folgenden Liganden TNFSF1 (LTalpha), TNFSF2 (TNF), TNFSF3 (LTbeta), TNFSF4 (OX40L), TNFSF5 (CD40L), TNFSF6 (FasL), TNFSF7 (CD27L), TNFSF8 (CD30L), TNFSF9 (4-1BBL), TNFSF10 (TRAIL), TNFSF11 (RANKL), TNFSF12 (TWEAK), TNFSF13 (APRIL), TNFSF13B (BLYS), TNFSF14 (LIGHT), TNFSF15 (VEGI), TNFSF16 (CD30L), TNFSF18 (AITRL) und EDA, die gleichfalls als Abschnitt (1) in einem erfindungsgemäßen Konstrukt dienen können. Insbesondere werden diesbezüglich alle membranständigen Typ2 -Proteine (C-terminus extracellulär), die eine trimere Organisation ihrer Untereinheiten als Voraussetzung für biologische Aktivität bedingen, mitoffenbart.

Der Linkerabschnitt (2) zwischen den Abschnitten (1) und (3) (Zytokin- bzw. Antikörper-Modul) stellt sich bei erfindungsgemäßen Polypeptidkonstrukten bspw. als eine flexible Verbindung dar, vorzugsweise jedoch ohne die intrinsischen Trimerisierungseigenschaften des betreffenden Zytokins negativ zu beeinflussen, wie in den Beispielskonstrukten (C), (D) und (F) (Linker-Aminosäuresequenz AAAVELE, s. Fig. 4) gezeigt. Vorzugsweise werden Linker mit intrinsischen Di- oder Multimerisierungseigenschaften (bspw. Tri- oder Hexamere) gewählt, bspw. um eine erhöhte Stabilität des multimeren Konstruktes zu erreichen, z.B. durch intrinsische Struktureigenschaften des Linkerpeptids wie coiled-coil Strukturen und/oder Ausbildung intermolekularer Disulfidbrücken mit dem Ergebnis kovalenter Verknüpfungen. In diesem Fall stellt sich der Linker (Abschnitt (2)) als Polymerisierungsmodul dar.

Im Falle eines Linkers, der als Dimerisierungsmodul (Linkertyp 2a) wirkt, wird beispielsweise eine Immunoglobulin hinge Region und CH3-Domäne eines humanen Immunglobulingens (AA 363-489, humanes IgG1, NCBI Accession No. AAF21613) bevorzugt (Linker im Beispielskonstrukt (A), s. Fig. 4). Ein Trimerisierungsmodul (Linkertyp 2b) als Linker kann bspw. aus einer Domäne des Tenascin-Moleküls (AA 110-139, Swiss Prot. Accession No. P10039, Huhn; oder Swiss Prot. Accession No. P24821,human) aufgebaut sein. Schließlich kann ein Linker als Hexamerisierungsmodul, also mit Hexamerisierungseigenschaften, beispielsweise eine im Vergleich zu Linkertyp 2b erweiterte Domäne des Tenascinmoleküls (AA 34-139, Swiss Prot. Accession No. P10039, Huhn; oder Swiss Prot. Accession No. P24821, human) aufweisen (Linkertyp 2c). In jedem Fall können die Sequenzen von nativen Polypeptiden oder Fragmenten dieser nativen Polypeptide, die als Linker in Abschnitt (2) eines erfindungsgemäßem Polypeptids zum Einsatz kommen, auch in Form biologisch aktiver Varianten derselben im Sinne dieser Erfindung und nach obiger Definition auftreten.

Alternativ sind in Abschnitt (2) andere, natürlich vorkommende oder synthetisch hergestellte Linker-Peptide denkbar. Grundsätzlich kann ein Linker einer nativen oder variierten (Teil)sequenz aller Organismen, vorzugsweise aus Vertebraten, insbesondere aus Säugetieren, vor allem aus dem Menschen, entsprechen. Ferner sind als Linker bspw. alle Sequenzabschnitte von Proteinen geeignet, die durch Ausbildung von Supersekundärstrukturen Di- oder Multimere generieren, z.B. "Coiled-Coil-Helices" oder typische Kollagen-artige Tripelhelices (z.B. CMP, COMP, Kollagen, Laminin). Auch Abschnitte von Proteinen aus der C1q-Familie oder von Collectinen sind typischerweise für eine Di- oder Multimerisierung geeignet. So etwa kann bspw. die extrazelluläre Domäne eines Mitglieds der TNF-Ligandenfamilie als Abschnitt (1) eines erfindungsgemäßen Polypeptids in Form eines Pentamers durch Rekombination mit den entsprechenden Pentamerisierungsdomänen von COMP als Linkerabschnitt (2) exprimiert werden. Erfindungsgemäß kann es sich um Homo- oder Heterodi- oder -multimere von erfindungsgemäßen Fusionsproteinen handeln.

Ein erfindungsgemäßes Polypeptid wird dann ganz besonders bevorzugt sein, wenn der Abschnitt (3) ein Antikörperfragment eines Säugetiers, insbesondere murinen, oder humanen Ursprungs, ist oder ein humanisiertes Antikörperfragment, bspw. mit Säugetierursprung, ist. Im Falle der Humanisierung besteht der Abschnitt (3) typischerweise aus einem nach dem Stand der Technik hergestellten scFv murinen, durch CDR grafting humanisierten oder vollständig humanen Ursprungs.

Der Abschnitt (3) eines erfindungsgemäßen Polypeptids wird vorzugsweise Spezifität für ein im Tumorgewebe selektiv bzw. dominant exprimiertes Antigen aufweisen. Dieses Tumorantigen kann prinzipiell auf den malignen Zellen selbst exprimiert sein oder auch im nichtmalignen Anteil des Tumors, den Stromazellen oder dem Tumorendothel. Derartige Antigene nichtmaligner Gewebeanteile eines soliden Tumors (Karzinoms) sind einerseits genetisch invariant, andererseits bei unterschiedlichsten Tumorentitäten vorkommend und damit universelle Tumormarker. Beispiele für der derartige Tumorantigene, gegen die ein Antikörperfragment des Abschnitts (3) eines erfindungsgemäßen Polypeptids gerichtet sein kann, sind der VEGFR bzw. der VEGFR/VEGF Komplex sowie das Integrin aᵥβ₃ und die Fibronektin Isoform ßFn als weitgehend selektive Zielstrukturen des Tumorendothels und das Fibroblast activation protein (FAP) als selektiver Marker des Tumorstromas. Alle vorgenannten Beispiele können mit spezifischen scFv wirksam erfasst werden, weswegen sich derartige scFv ("single chain Fv") besonders als Abschnitt (3) auf einem erfindungsgemäßen Antikörper eignen.

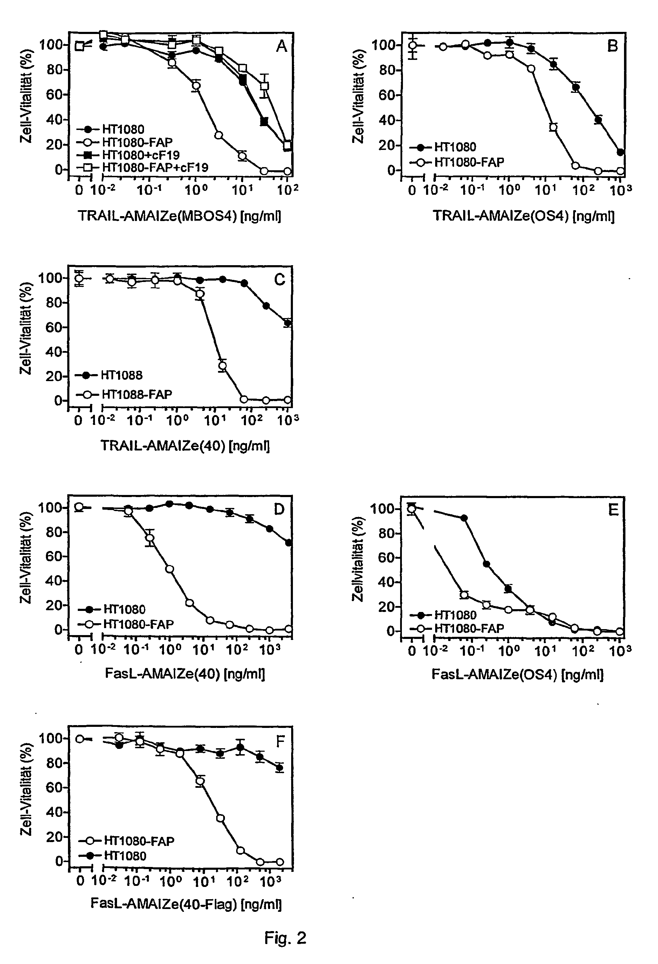

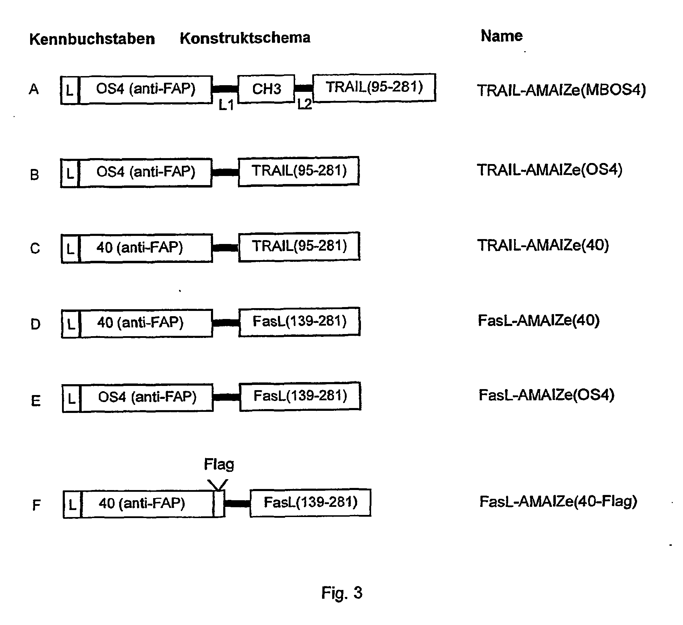

Damit ist ein bevorzugtes erfindungsgemäßes Polypeptid (Beispiele siehe Abb. 2 und 3) ein rekombinantes, homo-di- oder -trimeres Fusionsprotein prinzipiell enthaltend in einer definierten Abfolge der folgenden Strukturelemente (Monomer): (Abschnitt (3)) N-terminal ein murines, humanisiertes oder humanes Einzelkettenantikörperfragment (scFv) bestehend aus VH-peptid-linker-VL; Abschnitt (2) eine Linkersequenz ohne oder mit kovalenten Multimerisierungseigenschaften, z.B. Dimerisierungs- (2a), Trimerisierungs-(2b) oder Hexamerisierungs-Domäne (2c); (Abschnitt (1)) die humane extrazelluläre Domäne des TRAIL (AA 95-281, NCBI Accession No. U37518) oder des FasL (AA 139-281, NCBI Accession No U11821) C-terminal. Analog können bspw. CD40L oder andere Zytokinmitglieder der TNF-Familie als Abschnitt (1) entsprechender erfindungsgemäßer Polypeptide dienen.

Offenbart werden im Rahmen der vorliegenden Erfindung auch alle sich aus den erfindungsgemäßen Konstrukten durch spezifische Linkerwahl (2) ergebenden Di- oder Multimere, auf die sich die Gesamtoffenbarung zu erfindungsgemäßen Konstrukten inhaltsgleich bezieht. Insoweit fällt ein Di- oder Multimer von erfindungsgemäßen Polypeptiden nach Maßgabe der vorliegenden Offenbarung immer unter den weiteren Begriff "erfindungsgemäßes Polypeptid".

Ein weiterer Gegenstand der vorliegenden Erfindung sind DNA-Sequenzen, die für Fusionsproteine der vorgenannten erfindungsgemäßen Art kodieren (Nukleinsäurekonstrukte) oder einen solchen für ein erfindungsgemäßes Polypeptid codierenden Bereich enthalten. Derartige DNA-Sequenzen werden in Expressionsvektoren exprimiert, wobei auch die entsprechenden Expressionsvektoren, die eine DNA-Sequenz für die erfindungsgemäßen Fusionsproteine enthalten, Gegenstand der Erfindung sind. Vorzugsweise weisen erfindungsgemäße Vektoren die Fähigkeit zur Expression und/oder Amplifikation in einer prokaryontischen und/oder eukaryontischen Zelle auf, insbesondere betrifft die vorliegende Erfindung Plasmidvektoren, bspw. pBABEpuro, oder auch retrovirale Vektoren, insbesondere auch alle jene Vektorsysteme, die gentherapeutisch zur Anwendung kommen können, z.B. auch adenovirale Vektorsysteme. Im Rahmen der vorliegenden Erfindung werden damit auch gentherapeutische Verfahren mit erfindungsgemäßen Vektoren oder Nukleinsäurekonstrukten als Behandlungsmethode für die erfindungsgemäß offenbarten medizinischen Indikationen offenbart.

Weiterhin gehören zur vorliegenden Erfindung solche Wirtszellen, die mit DNA-Sequenzen (Nukleinsäurekonstrukte), die für die erfindungsgemäßen. Fusionsproteine kodieren, transfiziert sind. Ganz besonders bevorzugt sind in diesem Zusammenhang Wirtszellen, die mit erfindungsgemäßen Expressionsvektoren oder erfindungsgemäßen Nukleinsäurekonstrukten transfiziert sind, wobei die Expressionsvektoren wiederum DNA-Sequenzen enthalten, die für die erfindungsgemäßen Fusionsproteine kodieren.

Ein weiterer Gegenstand der vorliegenden Erfindung sind Verfahren zur Herstellung (Expression und isolierung) von erfindungsgemäßen Polypeptiden, wobei ein erfindungsgemäßes Isolierungsverfahren typischerweise gekennzeichnet ist durch (a) Bereitstellen eines erfindungsgemäßen Vektors oder eines Nukleinsäurekonstrukts, (b) Transfektion von Zellen mit einem gemäß Verfahrensschritt (a) erhaltenen Vektor oder Nukleinsäurekonstrukt, (c) Kultivierung der gemäß (b) transfizierten Zellen, und (d) Isolierung von unter entsprechenden Bedingungen exprimierten erfindungsgemäßen Polypeptiden aus den Wirtszellen und/oder dem Kulturüberstand. Die Expression des Fusionsproteins erfolgt hierbei typischerweise nach dem Stand der Technik in geeigneten Expressionssystemen, vorzugsweise als sezerniertes Produkt stabiler Transfektanten, z.B CHO-Zellen oder in anderen tierischen Zellen wie Cos7 oder SF9 (Insektenzellen) bzw. weiteren eukaryontischen Zellsystemen, z.B. *Pichia pastoris.* Vorzugsweise werden die exprimierten erfindungsgemäßen Polypeptide jeweilige zur Sekretion in dem Zellsystem geeignete Leadersequenzen aufweisen. Daher werden die zur Expression eingesetzten erfindungsgemäßen Vektoren auch codierende Abschnitte enthalten, die für eine funktionelle Leadersequenz codieren, z.B. wie in Brocks et al. (Immunotechnology 3:173-184, 1997) für Säuger und Insektenzellen beschrieben, bzw. pPICZalpha-Vektoren (INVITROGEN) zur Expression und Sekretion in der Hefe *Pichia pastoris.*

Erfindungsgemäße Polypeptide, ggf. aber auch Nukleinsäurekonstrukte, Vektoren oder Wirtszellen, (hier unter der Kategorie "erfindungsgemäße Substanzen" zusammengefaßt), kommen auch als Arzneimittel oder zur Herstellung eines Arzneimittels in Betracht. Ihre Verwendung ist insbesondere dann gegeben, wenn erfindungsgemäße Substanzen nach antikörpervermittelter Bindung des Fusionsproteins an ein spezifisches, zellmembranexprimiertes Zielmolekül die volle biologische Wirkung über den entsprechenden Zytokin-Rezeptor entfalten soll. Durch geeignete Auswahl der Antikörperspezifität wird die Zytokin-Aktivität der erfindungsgemäßen Substanz auf das zu behandelnde Gewebe, z.B. Tumorgewebe, gerichtet und es kann ein auf die jeweilige Indikation/Tumorentität spezifisch abgestimmtes/optimiertes Therapeutikum hergestellt werden. Ein erfindungsgemäßes Polypeptid wird z.B. bei Anwendung als Tumortherapeutikum, insbesondere zur Behandlung solider Tumore, aber auch lymphatischer Tumore (benigne oder maligne), nach in vivo Verabreichung durch den Antikörper-Anteil zunächst spezifisch im Tumorareal durch vom Tumor selbst oder das reaktive TumorstromalTumorgefäßsystem gebildete Membranmarker angereichert und dort Zytokin-Rezeptor-positiven Tumorzellen oder zytokinsensitiven Zellen des reaktiven tumorversorgenden Normalgewebes präsentiert.

Die Verwendung erfindungsgemäßer Substanzen ist aber grundsätzlich auch immer dann zur Anwendung im therapeutischen Bereich erwünscht, wenn die Aktivierung einer Signaltransduktionskette, bspw. die durch die TNF-Rezeptorfamilie ausgelösten Signalkaskaden, bspw. eine apoptotische Signalkaskade ausgelöst werden soll. Somit kommt die Verwendung erfindungsgemäßer Substanzen bei der Behandlung bzw. zur Herstellung eines Arzneimittels zur Behandlung aller hyperproliferativer Erkrankungen in Betracht, bspw. auch zur zielgerichteten Ausschaltung von Zellen des Immunsystems bei überschießenden Immunreaktionen, bspw. bei Autoimmunerkrankungen, wie z.B. multiple Sklerose, rheumatoide Arthritis, Diabetes mellitus und TEN, oder bei fehlgeleiteten Immunreaktionen gegen Fremdantigene, wie sie z.B. bei Infektionserkrankungen (bakteriell (bspw. durch Mykobakterien), viral oder protozoologisch) auftreten können. In Betracht kommt ferner die Behandlung von Stoffwechselerkrankungen oder allgemeinen hyperinflammatorischen Zuständen, insbesondere chronischen Entzündungen, bspw. auch bei Allergien, aber auch die Behandlung von Abstoßungsreaktionen des Immunsystems des Patienten gegen Fremdgewebe. In den vorgenannten Fällen muß jeweils der Antigenbindende Abschnitt (3) eines erfindungsgemäßen Polypeptids charakteristische Marker auf der Oberfläche der Zielzellen, bei denen vorzugsweise eine apoptotische Signalkaskade mit dem Ziel des Zelltods ausgelöst werden soll, erkennen. Im Falle der Behandlung nach Transplantation von Fremdgewebe werden also bspw. die körpereigenen für die Abstoßungsreaktion verantwortlichen Zellen des Immunsystems des Transplantionspatienten als Zielzellen dienen.

Erfindungsgemäße Gegenstände, wie Nukleinsäurekonstrukte, Expressionsvektoren oder Wirtszellen kommen - wie zuvor offenbart - gleichfalls als Arzneimittel bspw. zur Behandlung der vorgenannten Erkrankungen in Betracht. In diesem Fall werden vorzugsweise dem zu behandelnden Patienten zu transfizierende Zellen entnommen, diese in vitro mit erfindungsgemäßen Expressionsvektoren transfiziert, kultiviert und dann als Retransplantat in den Patienten überführt. Die Transfektion wird vorzugsweise durch Nukleinsäurekonstrukte oder Expressionsvektoren vorgenommen, die die Expression an einen regulierbaren Promotor koppeln. Das transfizierte Eigentransplantat kann lokal bspw. injiziert werden - abhängig von der spezifischen Erkrankung und den spezifischen Zielzellen. Lokale Verabreichung ist bspw. im Fall einer Tumortherapie bevorzugt. Hierbei werden Tumorzellen dem Patienten entnommen, in vitro transfiziert und dann, sofern möglich, direkt in den Tumor injiziert, bspw. zur Behandlung von Hauttumoren (z.B. Melanomen), Tumoren des Nervensystems (z.B. Glioblastomen).

Ein weiterer Gegenstand der vorliegenden Erfindung ist eine Zusammensetzung, enthaltend erfindungsgemäße Polypeptide, erfindungsgemäße Nukleinsäurekonstrukte, erfindungsgemäße Vektoren und/oder erfindungsgemäße Wirtszellen sowie pharmazeutisch unbedenkliche Hilfs-, Zusatz- und/oder Trägersubstanzen (z.B. auch Lösungsvermittler). Damit wird erfindungsgemäß eine Kombination erfindungsgemäßer Substanzen mit pharmazeutisch akzeptablen Träger-, Hilfs- und/oder Zusatzstoffen offenbart. Entsprechende Herstellungswege sind bei "Remington's Pharmaceutical Sciences" (Mack Pub. Co., Easton, PA, 1980) offenbart, das Bestandteil der Offenbarung der vorliegenden Erfindung ist. Für die parenterale Verabreichung kommen als Trägerstoffe bspw. steriles Wasser, sterile Kochsalzlösungen, Polyalkylenglykole, hydrogenierte Naphthalen und insbesondere biokompatible Lactidpolymere, Lactid/Glycolidcopolymer oder Polyoxyethylen-/Polyoxypropylencopolymere in Betracht. Derartige erfindungsgemäße Zusammensetzungen kommen für alle oben offenbarten medizinischen Indikationen in Betracht. Darüber hinaus können erfindungsgemäße Zusammensetzungen Füllsubstanzen oder Substanzen, wie Lactose, Mannitol, Substanzen zur kovalenten Anknüpfung von Polymeren, wie z.B. Polyethylenglykol an erfindungsgemäße Inhibitoren, Komplexierung mit Metallionen oder Einschluß von Materialien in oder auf besondere Präparationen von Polymerverbindung, wie z.B. Polylaktat, Polyglykolsäure, Hydrogel oder auf Liposomen, Mikroemulsion, Micellen, unilamelare oder multilamelare Vesikel, Erythrozyten-Fragmente oder Sphäroplasten, enthalten. Die jeweiligen Ausführungsformen der Zusammensetzungen werden abhängig vom physikalische Verhalten, beispielsweise in Hinblick auf die Löslichkeit, die Stabilität, Bioverfügbarkeit oder Abbaubarkeit gewählt. Konstrollierte oder konstante Freisetzung der erfindungsgemäßen Wirkstoffkomponente in der Zusammensetzung schließt Formulierungen auf der Basis lipophiler Depots ein (z.B. Fettsäuren, Wachse oder Öle). Im Rahmen der vorliegenden Erfindung werden auch Beschichtungen erfindungsgemäßer Substanzen oder Zusammensetzungen, enthaltend solche Substanzen, nämlich Beschichtungen mit Polymeren offenbart (z.B. Poloxamere oder Poloxamine). Weiterhin können erfindungsgemäßen Substanzen bzw. Zusammensetzungen protektive Beschichtungen, z.B. Proteaseinhibitoren oder Permeabilitätsverstärker, aufweisen.

Grundsätzlich werden im Rahmen der vorliegenden Erfindung für erfindungsgemäße Substanzen oder erfindungsgemäße Zusammensetzungen alle im Stand der Technik bekannten Verabreichungswege offenbart, bevorzugt erfolgt die Herstellung eines Arzneimittels zur Behandlung der vorgenannten Erkrankungen oder Störungen auf dem parenteralen, d.h. beispielsweise subkutanen, intramuskulären oder intravenösen, oder oralen oder intranasalen Verabreichungsweg. Typischerweise werden efindungsgemäße pharmazeutische Zusammensetzungen fest, flüssig oder aerosolartig (z. B. Spray) sein - je nach Art der Konfektionierung.

Zusammenfassend ist festzustellen, dass erfindungsgemäß Antikörper-Zytokin-Fusionsproteine mit proapoptotischen und immunmodulierenden Eigenschaften zur Verfügung gestellt werden, die lösliche Formen a priori membranständiger Zytokine enthalten. Durch die im erfindungsgemäßen Polypeptid vorhandene Antikörper-Funktion kann das im übrigen bioinaktive, respektive eingeschränkt aktive Zytokin durch Bindung an ein spezifisches, zellmembranständiges Zielmolekül die volle biologische Zytokin-Wirkung über den/die entsprechenden Zytokinrezeptor/en entfalten. Durch geeignete Auswahl der Antikörperspezifität wird die Zytokin-Aktivität auf das zu behandelnde Gewebe, z.B. Tumorgewebe, gerichtet und es kann ein auf die jeweilige Indikation/Tumorentität spezifisch abgestimmtes/optimiertes Therapeutikum hergestellt werden. Insgesamt wird somit die Selektivität der Zytokin-Wirkung mit den vorliegenden erfindungsgemäßen Polypeptiden durch zwei Mechanismen erreicht: Einerseits über die Antikörper-vermittelte, d.h. scFv, selektive Anreicherung des im nicht antigengebundenen Zustand inaktiven, respektive eingeschränkt aktiven Zytokins im Tumor und andererseits durch dessen ortsspezifische Aktivierung via Präsentation in ein voll signalfähiges, vor allem auch Apoptose-induzierendes Molekül.

Die vorliegende Erfindung wird durch die nachfolgenden Figuren näher erläutert.

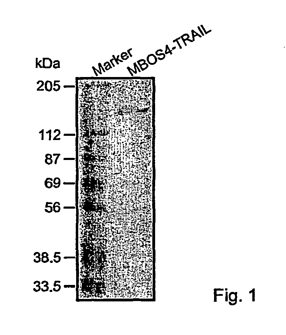

Figur 1 zeigt das Ergebnis einer gelelektrophoretischen Auftrennung nach der Expression eines erfindungsgemäßen Fusionsproteins (Struktur des Fusionsproteins s. Ausführungsbeispiel 1, TRAIL-AMAIZe(MBOS4), abgekürzt in Fig.1 als MBOS4-TRAIL). Die Western-Blot-Analyse zeigt, dass das Fusionsprotein unter nicht reduzierenden Bedingungen eine Bande bei ca. 140 kDa ergibt, was mit dem kalkulierten MW des CH3-verknüpften Dimers von 2 x 65 = 130 kDa gut übereinstimmt.

In Figur 2 sind die Ergebnisse von Untersuchungen in Hinblick auf die präferentielle Apoptoseinduktion durch Beispiele einiger erfindungsgemäßer AMAIZe-Polypeptide auf FAP-positiven Tumorzellen dargestellt. Figur 2 zeigt in allen ihren Bestandteilen (Figuren 2A bis 2F) eine Auftragung der Zellaktivität (in %) gegen die Konzentration der jeweils angegebenen AMAIZe-Proteine. Die in Figur 2A eingezeichneten Kurvenverläufe (Legende in Figur 2) geben die Ergebnisse der Behandlung von FAP-positiven (HT1080-FAP) oder FAP-negativen (HT 1080) Zellen mit TRAIL-AMAIZe(MBOS4) nach Vorinkubation mit dem FAP-spezifischen Antikörper cF19 bzw. ohne entsprechende Vorinkubation wieder.
In allen weiteren dargestellten Fällen (Fig. 2B-F) ist zu erkennen, daß die Zytotoxizität der verschiedenen AMAIZe-Konstrukte immer auf den Antigenexprimierenden Zellen (HT1080-FAP), d.h. den Zellen, auf denen die erfindungsgemäßen AMAIZe-Konstrukte Antikörper-vermittelt binden, größer ist als auf den entsprechenden Antigen-negativen parentalen Zellen (HT1080).
Die Abhängigkeit der gesteigerten Sensitivität FAP-exprimierender Zellen von der Bindung von erfindungsgemäßen AMAIZe-Konstrukten an FAP ist beispielhaft in Fig. 2A gezeigt Hier wird durch Kompetition eines FAP-spezifische Antikörpers cF19 (cF19, schwarze Quadrate) mit dem AMAIZe Konstrukt TRAIL-AMAIZe(MBOS4) um die Bindung an das zellexprimierte FAP die zytotoxische Wirkung eben dieses AMAIZe-Konstruktes auf den FAP-positiven Zellen auf ein Maß reduziert, wie es auch in FAP-negativen Zellen beobachtet wird. Auf FAP-negative Zellen hat die Zugabe von cF19 hingegen keinen Einfluß. Damit ist die Antikörper-vermittelte Spezifität durch kompetitive Hemmung der verstärkten Apoptose-Induktion über den FAP spezifischen monoklonalen Antikörper cF19 eindeutig belegt.

In Figur 3 sind Beispiele von erfindungsgemäßen AMAIZe-Konstrukten mit TRAIL und mit FasL als Zytokin-Modul, den unabhängigen FAP-spezifischen Antikörpern Klon OS4 und Klon 40 sowie verschiedenen Linkern zwischen Antikörper- und Zytokin-Modul dargestellt (erfindungsgemäße Konstrukte (A)-(F)). Alle erfindungsgemäßen Konstrukte besitzen die Eigenschaft der antigenabhängigen Induktion/Verstärkung von Apoptose. Die hergestellten Konstrukte sind im folgenden schematisch dargestellt. Ihre spezifische AMAIZe-Aktivität (präferentielle Apoptoseinduktion auf antigen-positiven Zellen) findet sich in Figur 2 beschrieben. Der für die AS-Sequenzen vorliegendenfalls gewählte Code ist der Ein-Buchstaben-Code). Abschnitt (2) (der Linker) stellt die Verbindung zwischen dem Abschnitt (3) und dem Zytokin-Anteil (1) (bspw. TRAIL oder FasL in den dargestellten Konstrukten) im erfindungsgemäßen Molekül her und gewährleistet, im Falle des Einsatzes von speziellen Linkern bspw. des Typs 2a, 2b oder 2c, gleichzeitig die kovalente Verknüpfung des Fusionsproteins während der Biogenese.

### Konstrukt (A): TRAIL-AMAIZe(MBOS4)

NH₂-[Leader]-[OS4-VHIVL]-[Linker1]-[CH3]-[Linker2]-[TRAIL(95-281)]-COOH

| | |
|---|---|
| OS4-VH/VL: | FAP-spezifisches humanes "single chain"-Antikörperfragment |
| CH3: | CH3-Domäne (AA 363-489) eines humanen IgG1 |
| Linker1: | "hinge"-Region eines humanen IgG1 (**Fettdruck**) mit einem C-terminalen poly Gly-Linker (kursiv) |
| | (**RTVAAPSVFAVFAAAVEPKSCDKTHTCPPC***GGGSSGGG SG*) |
| Linker2: | poly Gly-Linker (GGGGTGGGS) |
| TRAIL(95-281): | extrazelluläre Domäne des humanen TRAIL (AA 95-281) |

Der Linker des Konstrukts (A): TRAIL-AMAIZe(MBOS4) besitzt Dimerisierungseigenschaften.

### Konstrukt (B): TRAIL-AMAIZe(OS4)

NH₂-[Leader]-[OS4-VH/VL]-[Linker]-[TRAIL(95-281)]-COOH

| | |
|---|---|
| OS4-VH/VL: | FAP-spezifisches humanes "single chain =Antikörperfragment |
| Linker: | RTVAAPSVFAVFAAAVELE |
| TRAIL(95-281): | extrazelluläre Domäne des humanen TRAIL (AA 95-281) |

### Konstrukt (C): TRAIL-AMAIZe(40)

NH₂-[Leader]-[40-VH/VL]-[Linker]-[TRAIL(95-281)]-COOH

| | |
|---|---|
| 40-VH/VL: | FAP-spezifisches humanes "single chain"-Antikörperfragment |
| Linker: | AAAVELE |
| TRAIL(95-281): | extrazelluläre Domäne des humanen TRAIL (AA 95-281) |

### Konstrukt (D): FasL-AMAIZe(40)

NH₂-[Leader]-[40-VH/VL]-[Linker]-[FasL(139-281)]-COOH

| | |
|---|---|
| 40-VHNL: | FAP-spezifisches humanes "single chain"-Antikörperfragment |
| Linker: | AAAVELE |
| FasL(139-281): | extrazelluläre Domäne des humanen FasL (AA 139-281) |

### Konstrukt (E): FasL-AMAIZe(OS4)

NH₂-[Leader]-[OS4-VH/VL]-[Linker]-[FasL(139-281)]-COOH

| | |
|---|---|
| OS4-VH/VL: | FAP-spezifisches humanes "single chain"-Antikörperfragment |
| Linker: | RTVAAPSVFAVFAAA |
| FasL(139-281): | extrazelluläre Domäne des humanen FasL (AA 139-281) |

### Konstrukt (F): FasL-AMAlZe(40-Flag)

NH₂-[Leader]-[40-VHNL]-[Flag-tag-][Linker]-[FasL(139-281)]-COOH

| | |
|---|---|
| 40-VH/VL: | FAP-spezifisches humanes "single chain"-Antikörperfragment |
| Flag-tag: | DYKDDDDK |
| Linker: | AAAVELE |
| FasL(139-281): | extrazelluläre Domäne des humanen FasL (AA 139-281) |

Die vorliegende Erfindung wird durch die nachfolgenden Ausführungsbeispiele näher erläutert.

### Beispiel 1

### Expression eines erfindungsgemäßen Fusionsproteins

Es wurde ein erfindungsgemäßes Fusionsprotein in CHO-DG44-Zellen exprimiert. Bei diesem Fusionsprotein handelt es sich um TRAIL-AMAIZe(MBOS4), abk. MBOS4-TRAIL in Fig.1, (kovalentes Dimer) mit nachfolgender Struktur (s. auch Figur 3):
NH₂-[Leader]-[OS4-VH/VL]-[Linker1]-[CH3]-[Linker2]-[TRAIL(95-281)]-COOH

| | |
|---|---|
| OS4-VH/VL: | FAP-spezifisches humanes "single chain"-Antikörperfragment |
| CH3: | CH3-Domäne (AA 363-489) eines humanen IgG1 |
| Linker1: | "hinge"-Region eines humanen IgG1 (b) mit einem C-terminalen poly Gly-Linker (kursiv) |
| | (**RTVAAPSVFAVFAAAVEPKSCDKTHTCPP***CGGGSSGGGSG*) |
| Linker2: | poly Gly-Linker (GGGGTGGGS) |
| TRAIL(95-281): | extrazelluläre Domäne des humanen TRAIL (AA 95-281) |

### Beispiel 2

### Konstruktion der erfindungsgemäßen Polypeptide FasL-AMAIZe(40) und TRAIL-AMAIZe(40)

Die Fusionsproteine wurden, wie folgt, hergestellt:
1. Das single chain Antikörper Fragment (scFv) Nr. 40 (im weiteren als 40 bezeichnet) wurde nach Standardmethoden aufgrund der Bindung an FAP aus einer scFv-Phagen Expressionsbank, die in dem Vektor pSEX (siehe Brocks et al, Molecular Medicine 7:461-469; Mersmann et al, Int.J. Cancer 92:240-248) vorlag, isoliert.
2. Der scFv 40 wurde mittels Pvu2 und Not1 aus pSEX ausgeschnitten und in die entsprechenden sites des Minibody-Konstrukts pW6 (Wüest, T., Dissertation Uni Stuttgart, 2001) eingesetzt. Hierzu wurde dem Plasmid pW6 zuvor der zwischen diesen Stellen liegende DNA-Bereich durch entsprechenden Restriktionsverdau und präparativer Agarosegel-Elektrophorese nebst DNA-Elution entfernt Durch diesen Klonierungsschritt wurde der scFv 40 so zwischen eine eukaryotische Ig Leadersequenz (upstream von 40) und der konstanten Region (Fc Region) eines humanen Antikörpers (IgG1, downstream von 40)) kloniert, daß die Expression eines sekretierbaren, divalenten Minibodies, wie bei Hu et al (Cancer Research 56:3055) beschrieben, möglich ist.
3. Leader + scFv 40 + Fc wurden durch proof-reading PCR mit den Primern 1 und 2 amplifiziert und das scFv Fragment mittels der durch den Primer 1 eingeführten Kpn1-Schnittstelle und der zwischen scFv 40 und Fc-Region liegenden Not1-Schnittstelle in die entsprechenden Schnittstellen des eukaryontischen Expressionsvektors pcDNA3.1 (Invitrogen) eingeführt.

4a. Zur Fertigstellung von FasL-AMAIZe(40) wurden die AS 139 bis 281 + Stopcodon von humanem FasL mittels der Primer 3 und 4 und proof-reading PCR amplifiziert und in die Schnittstellen Not1 und Xba1 des in 3. erhaltenen pcDNA3.1-Klonierungsintermediates eingesetzt. In das FasL-Amplikon wurden hierzu durch die verwendeten Primer eine Not1 und eine Nhe1-Schnittstelle, die mit Xba1 kompatibel ist, eingefügt. Das so erhaltene fertige Konstrukt erlaubt die Expression des Fusionsproteins FasL-AMAIZe(40).
4b. Zur Fertigstellung von TRAIL-AMAIZe(40) wurden die AS 95 bis 281 + Stopcodon von humanem TRAIL mittels den Primern 5 und 6 und proof-reading PCR amplifiziert und in die Schnittstellen Not1 und Xba1 des in 3. erhaltenen pcDNA3.1-Klonierungsintermediates eingesetzt. In das TRAIL-Amplikon wurden hierzu durch die verwendeten Primer eine Not1 und eine Xba1-Schnittstelle eingefügt. Das so erhaltene fertige Konstrukt erlaubt die Expression des Fusionsprotein TRAIL-AMAIZe(40).
5. Zur Gewinnung von TRAIL-AMAIZe(40) bzw. FasL-AMAIZe(40) wurden HEK293 Zellen mit den unter 4. beschriebenen Konstrukten nach Angaben des Herstellers mit Lipofektamin (Gibco-BRL) transfiziert. 48 Stunden nach Transfektion wurden die AMAIZe-Konstrukt-Überstände sterilfiltriert und bei 4°C bis zur weiteren Verwendung gelagert.

Alle Klonierungs- und PCR-Amplifikationsschritte erfolgten nach üblichen Standardprozeduren mit den nachfolgenden Primern. Alle Konstrukte wurden zur Verifikation der cDNA-Sequenz sequenziert.
Primer 1
   5'CGG GGT ACC TCG ACC ATG GAC TGG ACC TGG CGC GTG 3'
Primer 2
   5'CCG GAA TTC CAC AGC CAG GTG CAA CTA GTT GAG CC 3'
Primer 3 
5'CTA GGT GCG GCC GCA GTT GAG CTC GAG GAA AAA AAG GAG CTG
AGG AAA GTG 3'
Primer 4
   5'CTA GCT AGC GTG CTT CTC TTA GAG CTT ATA TAA GCC 3'
Primer 5 
5'GTC TTC GCG GCC GCA GTT GAG CTC GAG ACC TCT GAG GAA ACC ATT
TCT ACA G 3'
Primer 6
   5'TGC TCT AGA CCA GGT CAG TTA GCC AAC TAA AAA GGC 3'

### Beispiel 3

Nachweis der Antigen-abhängigen Aktivierung am Beispiel des FAP-spezifischen TRAIL-AMAIZe(MBOS4) (siehe auch Fig. 2A).

TRAIL-AMAIZe(MBOS4) wurde - analog wie in Beispiel 2 beschrieben - bereitgestellt.

Anschließend wurden FAP-positive (HT1080-FAP) und FAP-negative (HT1080) Zellen mit dem FAP-spezifischen Antikörper cF19 vorinkubiert (1 h) oder blieben unbehandelt. Die Zellen wurden über Nacht in Gegenwart von CHX (2.5 µg/ml) mit den angegebenen Konzentrationen an TRAIL-AMAIZe(MBOS4) inkubiert. Die Quantifizierung der überlebenden Zellen erfogte mittels Kristallviolett-Färbung. In Figur 2A ist die Wirkung des TRAIL-AMAIZe(MBOS4) auf Zielzellen, die spezifisch vom Antikörper-Anteil des Fusionsproteins erkannt werden (FAP positive HT 1080) dargestellt.

### Beispiel 4

Präferentielle Apoptoseinduktion durch TRAIL-AMAIZe und FasL-AMAIZe auf FAP-positiven Tumorzellen (siehe auch Fig. 2A-F).

15 x 10³ HT1080 oder HT1080-FAP Zellen pro weil einer 96-well Platte wurden über Nacht kultiviert. Am nächsten Tag wurden die Zellen mit den angegebenen Mengen der verschiedenen Konstrukte für weitere 14-18 Stunden in der Anwesenheit von 2.5 µg/ml CHX (zur Sensibilisierung der Zellen für die Induktion von Todesrezeptor-vermittelter Apoptose) behandelt. Dann wurde abschließend die Vitalität der Zellen durch Färbung mit Kristall-Violett bestimmt. Die jeweiligen Werte für unbehandelte Gruppen lag in allen Fällen zwischen 700 und 850 mOD. Kontrollgruppen, in denen alle Zellen dem Zelltod anheimfielen, wiesen Werte von 100 bis 150 mOD auf. Zelltod der entsprechenden Positiv-Kontrollgruppen wurde durch sekundäres Quervemetzen eines löslichen, Flag-markierten FasL-Konstruktes (500 ng/ml) mittels des Flag-spezifischen Antikörpers M2 (Sigma) erreicht. Auch in diesem Fall wurden den Kulturen 2.5 µg CHX zugegeben.

### SEQUENZPROTOKOLL

**ANMELDER**
   NAME: Prof. Dr. Klaus Pfizenmaier
   STRASSE: Seehausstraße 7
   ORT: Tiefenbronn
   POSTLEITZAHL: 75233
   TELEFON: 07 11/6 85 69 86
   TELEFAX: 07 11/6 85 74 84
   AKTENZEICHEN:
**BEZEICHNUNG DER ERFINDUNG:**
   Ortsspezifische, antikörpervermittelte Aktivierung proapoptotischer Zytokine:
   AMAIZe (Antibody-Mediated Apoptosis Inducing Zytokine)
**ANZAHL DER SEQUENZEN:** 6 DNA Sequenzen
   6 Proteinsequenzen
**COMPUTERLESBARE FASSUNG:**
   DATENTRÄGER: Diskette
   COMPUTER: PC
   BETRIEBSSYSTEM: MS DOS
   SOFTWARE: Windows NT
**Sequenzprotokoll zu Patentanmeldung AMAIZe**
   **AZ#**
   **Anmeldernummer:**
**Sequenzbeschreibung 1:** Kodierende DNA Sequenz (unten, nur kodierender DNA Strang, Nukleotid (NT) 1-1845)und translatierte Aminosäuresequenz (oben, Einzelbuchstaben Kodierung der Aminosäuren (AA) 1-614)eines erfindungsgemäßen Antikörper-Zytokin-Fusionsproteins AMAIZe am Beispiel von TRAIL-AMAIZe(MBOS4) (Konstrukt A). 
1 M D W T W R V F C L L A V A P G 16
1 ATG GAC TGG ACC TGG CGC GTG TTT TGC CTG CTC GCC GTG GCT CCT GGG 48

17 A H S Q V Q L V Q S G A E V K K 32
49 GCC CAC AGC CAG GTG CAA CTA GTG CAG TCC GGC GCC GAA GTG AAG AAA 96

33 P G A S V K V S C K T S R Y T F 48
97 CCC GGT GCT TCC GTG AAA GTC AGC TGT AAA ACT AGT AGA TAC ACC TTC 144

49 T E Y T I H W V R Q A P G Q R L 64
145 ACT GAA TAC ACC ATA CAC TGG GTT AGA CAG GCC CCT GGC CAA AGG CTG 192

65 E W I G G I N P N N G I P N Y N 80
193 GAG TGG ATA GGA GGT ATT AAT CCT AAC AAT GGT ATT CCT AAC TAC AAC 240

81 Q K F K G R V T I T V D T S A S 96
241 CAG AAG TTC AAG GGC CGG GTC ACC ATC ACC GTA GAC ACC TCT GCC AGC 288

97 T A Y M E L S S L R S E D T A V 112
289 ACC GCC TAC ATG GAA CTG TCC AGC CTG CGC TCC GAG GAC ACT GCA GTC 336

113 Y Y C A R R R I A Y G Y D E G H 128
337 TAC TAC TGC GCC AGA AGA AGA ATC GCC TAT GGT TAC GAC GAG GGC CAT 384

129 A M D Y W G Q G T L V T V S S A 144
385 GCT ATG GAC TAC TGG GGT CAA GGA ACC CTT GTC ACC GTC TCC TCA GCC 432

145 S T K G P K L E E G E F S E A R 160
433 TCC ACC AAG GGC CCA AAG CTT GAA GAA GGT GAA TTT TCA GAA GCA CGC 480

161 V D I V M T Q S P D S L A V S L 176
481 GTA GAC ATT GTG ATG ACC CAA TCT CCA GAC TCT TTG GCT GTG TCT CTA 528

177 G E R A T I N C K S S Q S L L Y 192
529 GGG GAG AGG GCC ACC ATC AAC TGC AAG TCC AGT CAG AGC CTT TTA TAT 576

193 S R N Q K N Y L A W Y Q Q K P G 208
577 TCT AGA AAT CAA AAG AAC TAC TTG GCC TGG TAT CAG CAG AAA CCA GGA 624

209 Q P P K L L I F W A S T R E S G 224
625 CAG CCA CCC AAA CTC CTC ATC TTT TGG GCT AGC ACT AGG GAA TCT GGG 672

225 V P D R F S G S G F G T D F T L 240
673 GTA CCT GAT AGG TTC AGT GGC AGT GGG TTT GGG ACA GAC TTC ACC CTC 720
 
241 T I S S L Q A E D V A V Y Y C Q 256
721 ACC ATT AGC AGC CTG CAG GCT GAA GAT GTG GCA GTT TAT TAC TGT CAG 768

257 Q Y F S Y P L T F G Q G T K V E 272
769 CAA TAT TTT AGC TAT CCG CTC ACG TTC GGA CAA GGG ACC AAG GTG GAA 816

273 I K R T V A A P S V F A V F A A 288
817 ATA AAA CGT ACT GTG GCT GCA CCA TCT GTC TTC GCT GTC TTC GCG GCC 864

289 A V E P K S C D K T H T C P P C 304
865 GCA GTT GAG CCC AAA TCT TGT GAC AAA ACT CAC ACA TGC CCA CCA TGC 912

305 G G G S S G G G S G G Q P R E P 320
913 GGA GGA GGA AGT AGC GGA GGA GGA TCA GGA GGG CAG CCC CGA GAA CCA 960

321 Q V Y T L P P S R E E M T K N Q 336
961 CAG GTG TAC ACC CTG CCC CCA TCC CGG GAG GAG ATG ACC AAG AAC CAG 1008

337 V S L T C L V K G F Y P S D I A 352
1009 GTC AGC CTG ACC TGC CTG GTC AAA GGC TTC TAT CCC AGC GAC ATC GCC 1056

353 V E W E S N G Q P E N N Y K T T 368
1057 GTG GAG TGG GAG AGC AAT GGG CAG CCG GAG AAC AAC TAC AAG ACC ACG 1104

369 P P V L D S D G S F F L Y S K L 384
1105 CCT CCC GTG CTG GAC TCC GAC GGC TCC TTC TTC CTC TAT AGC AAG CTC 1152

385 T V D K S R W Q Q G N V F S C S 400
1153 ACC GTG GAC AAG AGC AGG TGG CAG CAG GGG AAC GTC TTC TCA TGC TCC 1200

401 V M H E A L H N H Y T Q K S L S 416
1201 GTG ATG CAT GAG GCT CTG CAC AAC CAC TAC ACG CAG AAG AGC CTC TCC 1248

417 L S G G G G T G G G S T S E E T 432
1249 CTG TCC GGA GGT GGC GGT ACC GGA GGT GGG TCT ACC TCT GAG GAA ACC 1296

433 I S T V Q E K Q Q N I S P L V R 448
1297 ATT TCT ACA GTT CAA GAA AAG CAA CAA AAT ATT TCT CCC CTA GTG AGA 1344

449 E R G P Q R V A A H I T G T R G 464
1345 GAA AGA GGT CCT CAG AGA GTA GCA GCT CAC ATA ACT GGG ACC AGA GGA 1392

465 R S N T L S S P N S K N E K A L 480
1393 AGA AGC AAC ACA TTG TCT TCT CCA AAC TCC AAG AAT GAA AAG GCT CTG 1440

481 G R K I N S W E S S R S G H S F 496
1441 GGC CGC AAA ATA AAC TCC TGG GAA TCA TCA AGG AGT GGG CAT TCA TTC 1488

497 L S N L H L R N G E L V I H E K 512
1489 CTG AGC AAC TTG CAC TTG AGG AAT GGT GAA CTG GTC ATC CAT GAA AAA 1536

513 G F Y Y I Y S Q T Y F R F Q E E 528
1537 GGG TTT TAC TAC ATC TAT TCC CAA ACA TAC TTT CGA TTT CAG GAG GAA 1584

529 I K E N T K N D K Q M V Q Y I Y 544
1585 ATA AAA GAA AAC ACA AAG AAC GAC AAA CAA ATG GTC CAA TAT ATT TAC 1632

545 K Y T S Y P D P I L L M K S A R 560
1633 AAA TAC ACA AGT TAT CCT GAC CCT ATA TTG TTG ATG AAA AGT GCT AGA 1680

561 N S C W S K D A E Y G L Y S I Y 576
1681 AAT AGT TGT TGG TCT AAA GAT GCA GAA TAT GGA CTC TAT TCC ATC TAT 1728

577 Q G G I F E L K E N D R I F V S 592
1729 CAA GGG GGA ATA TTT GAG CTT AAG GAA AAT GAC AGA ATT TTT GTT TCT 1776
 
593 V T N E H L I D M D H E A S F F 608
1777 GTA ACA AAT GAG CAC TTG ATA GAC ATG GAC CAT GAA GCC AGT TTT TTC 1824

609 G A F L V G * 615
1825 GGG GCC TTT TTA GTT GGC TAA 1845
**Merkmale Konstrukt A:**
Leitpeptidsequenz: NT 1-57, AA 1-19
Sequenz des Einzelketten (scFv)-Antikörperfragmentes OS4 (spezifisch für das Tumorstroma-Antigen FAP): NT 58-822, AA 20-274
Sequenz des Linkers 1 (L1) Zwischen scFv und Immunoglobulin-CH3 Domäne: NT 823-942, AA 275-314
Sequenz der Immunoglobulin-CH3 Domäne: NT 943-1254, AA 315-418
Sequenz des Linkers 2 zwischen Ig CH3 Domäne und TRAIL: NT 1254-1281, AA 419-427
Sequenz des humanen TRAIL Fragmentes (extrazelluläre Domäne, ab AA 95-281 des natürlichen, humanen TRAIL Moleküls): NT 1282-1842, AA 428-614
Stop Kodon: NT 1843-1845.
**Sequenzprotokoll zu Patentanmeldung AMAIZe**
   **AZ#**
   **Anmeldernummer:**
**Sequenzbeschreibung 2:** Kodierende DNA Sequenz (unten, nur kodierender DNA Strang, Nukleotid (NT) 1-1443)und translatierte Aminosäuresequenz (oben, Einzelbuchstaben Kodierung der Aminosäuren (AA) 1-480)eines erfindungsgemäßen Antikörper-Zytokin-Fusionsproteins AMAIZe am Beispiel von TRAIL-AMAIZe(OS4)(Konstrukt B). 
1 M D W T W R V F C L L A V A P G 16
1 ATG GAC TGG ACC TGG CGC GTG TTT TGC CTG CTC GCC GTG GCT CCT GGG 48

17 A H S Q V Q L V Q S G A E V K K 32
49 GCC CAC AGC CAG GTG CAA CTA GTG CAG TCC GGC GCC GAA GTG AAG AAA 96

33 P G A S V K V S C K T S R Y T F 48
97 CCC GGT GCT TCC GTG AAA GTC AGC TGT AAA ACT AGT AGA TAC ACC TTC 144

49 T E Y T I H W V R Q A P G Q R L 64
145 ACT GAA TAC ACC ATA CAC TGG GTT AGA CAG GCC CCT GGC CAA AGG CTG 192

65 E W I G G I N P N N G I P N Y N 80
193 GAG TGG ATA GGA GGT ATT AAT CCT AAC AAT GGT ATT CCT AAC TAC AAC 240

81 Q K F K G R V T I T V D T S A S 96
241 CAG AAG TTC AAG GGC CGG GTC ACC ATC ACC GTA GAC ACC TCT GCC AGC 288

97 T A Y M E L S S L R S E D T A V 112
289 ACC GCC TAC ATG GAA CTG TCC AGC CTG CGC TCC GAG GAC ACT GCA GTC 336

113 Y Y C A R R R I A Y G Y D E G H 128
337 TAC TAC TGC GCC AGA AGA AGA ATC GCC TAT GGT TAC GAC GAG GGC CAT 384

129 A M D Y W G Q G T L V T V S S A 144
385 GCT ATG GAC TAC TGG GGT CAA GGA ACC CTT GTC ACC GTC TCC TCA GCC 432

145 S T K G P K L E E G E F S E A R 160
433 TCC ACC AAG GGC CCA AAG CTT GAA GAA GGT GAA TTT TCA GAA GCA CGC 480

161 V D I V M T Q S P D S L A V S L 176
481 GTA GAC ATT GTG ATG ACC CAA TCT CCA GAC TCT TTG GCT GTG TCT CTA 528

177 G E R A T I N C K S S Q S L L Y 192
529 GGG GAG AGG GCC ACC ATC AAC TGC AAG TCC AGT CAG AGC CTT TTA TAT 576

193 S R N Q K N Y L A W Y Q Q K P G 208
577 TCT AGA AAT CAA AAG AAC TAC TTG GCC TGG TAT CAG CAG AAA CCA GGA 624

209 Q P P K L L I F W A S T R E S G 224
625 CAG CCA CCC AAA CTC CTC ATC TTT TGG GCT AGC ACT AGG GAA TCT GGG 672

225 V P D R F S G S G F G T D F T L 240
673 GTA CCT GAT AGG TTC AGT GGC AGT GGG TTT GGG ACA GAC TTC ACC CTC 720

241 T I S S L Q A E D V A V Y Y C Q 256
721 ACC ATT AGC AGC CTG CAG GCT GAA GAT GTG GCA GTT TAT TAC TGT CAG 768

257 Q Y F S Y P L T F G Q G T K V E 272
769 CAA TAT TTT AGC TAT CCG CTC ACG TTC GGA CAA GGG ACC AAG GTG GAA 816
 
273 I K R T V A A P S V F A V F A A 288
817 ATA AAA CGT ACT GTG GCT GCA CCA TCT GTC TTC GCT GTC TTC GCG GCC 864

289 A V E L E T S E E T I S T V Q E 304
865 GCA GTT GAG CTC GAG ACC TCT GAG GAA ACC ATT TCT ACA GTT CAA GAA 912

305 K Q Q N I S P L V R E R G P Q R 320
913 AAG CAA CAA AAT ATT TCT CCC CTA GTG AGA GAA AGA GGT CCT CAG AGA 960

321 V A A H I T G T R G R S N T L S 336
961 GTA GCA GCT CAC ATA ACT GGG ACC AGA GGA AGA AGC AAC ACA TTG TCT 1008

337 S P N S K N E K A L G R K I N S 352
1009 TCT CCA AAC TCC AAG AAT GAA AAG GCT CTG GGC CGC AAA ATA AAC TCC 1056

353 W E S S R S G H S F L S N L H L 368
1057 TGG GAA TCA TCA AGG AGT GGG CAT TCA TTC CTG AGC AAC TTG CAC TTG 1104

369 R N G E L V I H E K G F Y Y I Y 384
1105 AGG AAT GGT GAA CTG GTC ATC CAT GAA AAA GGG TTT TAC TAC ATC TAT 1152

385 S Q T Y F R F Q E E I K E N T K 400
1153 TCC CAA ACA TAC TTT CGA TTT CAG GAG GAA ATA AAA GAA AAC ACA AAG 1200

401 N D K Q M V Q Y I Y K Y T S Y P 416
1201 AAC GAC AAA CAA ATG GTC CAA TAT ATT TAC AAA TAC ACA AGT TAT CCT 1248

417 D P I L L M K S A R N S C W S K 432
1249 GAC CCT ATA TTG TTG ATG AAA AGT GCT AGA AAT AGT TGT TGG TCT AAA 1296

433 D A E Y G L Y S I Y Q G G I F E 448
1297 GAT GCA GAA TAT GGA CTC TAT TCC ATC TAT CAA GGG GGA ATA TTT GAG 1344

449 L K E N D R I F V S V T N E H L 464
1345 CTT AAG GAA AAT GAC AGA ATT TTT GTT TCT GTA ACA AAT GAG CAC TTG 1392

465 I D M D H E A S F F G A F L V G 480
1393 ATA GAC ATG GAC CAT GAA GCC AGT TTT TTC GGG GCC TTT TTA GTT GGC 1440

481 * 481
1441 TAA 1443
**Merkmale Konstrukt B:**
Leitpeptidsequenz: NT 1-57, AA 1-19
Sequenz des Einzelketten (scFv)-Antikörperfragmentes OS4 spezifisch für das Tumorstroma-Antigen FAP: NT 58-822, AA 20-274
Sequenz des Linkers zwischen scFv und TRAIL-Fragment (AA 95-281): NT 823-879, AA 275-293
Sequenz des humanen TRAIL Fragmentes (extrazelluläre Domäne, AA 95-281 des natürlichen, humanen TRAIL Moleküls): NT 880-1440 AA 294-480
Stop Kodon: NT 1441-1443.
**Sequenzprotokoll zu Patentanmeldung AMAIZe**
   **AZ#**
   **Anmeldernummer:**
**Sequenzbeschreibung 3:** Kodierende DNA Sequenz (unten, nur kodierender DNA Strang, Nukleotid (NT) 1-1386)und translatierte Aminosäuresequenz (oben, Einzelbuchstaben Kodierung der Aminosäuren (AA) 1-461)eines erfindungsgemäßen Antikörper-Zytokin-Fusionsproteins AMAIZe am Beispiel von TRAIL-AMAIZe(40)(Konstrukt C). 
1 M D W T W R V F C L L A V A P G 16
1 ATG GAC TGG ACC TGG CGC GTG TTT TGC CTG CTC GCC GTG GCT CCT GGG 48

17 A H S Q V Q L V Q S G G G M V E 32
49 GCC CAC AGC CAG GTA CAG CTG GTG CAG TCT GGG GGA GGC ATG GTA GAG 96

33 P G G S L R L S C A A S G F T F 48
97 CCT GGG GGG TCC CTT AGA CTC TCC TGT GCA GCC TCT GGA TTC ACT TTC 144

49 S N A W M S W V R Q A P G K G L 64
145 AGT AAT GCC TGG ATG AGC TGG GTC CGC CAG GCT CCA GGG AAG GGG CTG 192

65 E W V G R I K S K A G G G T A E 80
193 GAG TGG GTT GGC CGT ATA AAA AGC AAA GCT GGT GGT GGG ACA GCA GAG 240

81 Y A A P V K G R F T I S R D D S 96
241 TAC GCT GCA CCC GTG AAA GGC AGA TTC ACC ATC TCA AGA GAT GAT TCA 288

97 Q N T L Y L Q M N S L K T D D T 112
289 CAA AAC ACG CTG TAT CTG CAA ATG AAC AGC CTG AAA ACC GAC GAC ACA 336

113 A V Y Y C T T H V Y G A P R N W 128
337 GCC GTG TAT TAC TGT ACC ACA CAT GTC TAC GGT GCC CCC CGG AAC TGG 384

129 G Q G S L V T V S S A S T K G P 144
385 GGC CAG GGA TCC CTG GTC ACC GTC TCC TCA GCC TCC ACC AAG GGC CCA 432

145 K L E E G E F S E A R V Q S V L 160
433 AAG CTT GAA GAA GGT GAA TTT TCA GAA GCA CGC GTA CAG TCT GTG TTG 480

161 T Q P P S V S A A P G Q K V T I 176
481 ACT CAG CCG CCC TCA GTG TCT GCG GCC CCA GGA CAG AAG GTC ACC ATC 528

177 S C S G S S S N I G N N Y V S W 192
529 TCC TGC TCT GGA AGC AGC TCC AAC ATT GGA AAT AAT TAT GTC TCC TGG 576

193 Y V Q L P G T A P K L L I Y D N 208
577 TAC GTT CAA CTC CCA GGA ACA GCC CCC AAA CTC CTC ATT TAT GAC AAT 624

209 N K R F S G V P D R F S G S K S 224
625 AAT AAG CGA TTC TCA GGA GTT CCT GAC CGA TTC TCT GGC TCC AAG TCT 672

225 G T S A T L G I T G L Q T G D E 240
673 GGC ACG TCA GCC ACC CTG GGC ATC ACC GGG CTC CAG ACT GGG GAC GAG 720

241 A D Y Y C G A W D G S L R E A V 256
721 GCC GAT TAT TAC TGC GGA GCA TGG GAT GGC AGC CTG CGT GAA GCG GTA 768

257 F G G G T K V T V L G A A A V E 272
769 TTC GGC GGA GGG ACC AAG GTC ACC GTC CTA GGT GCG GCC GCA GTT GAG 816
 
273 L E T S E E T I S T V Q E K Q Q 288
817 CTC GAG ACC TCT GAG GAA ACC ATT TCT ACA GTT CAA GAA AAG CAA CAA 864

289 N I S P L V R E R G P Q R V A A 304
865 AAT ATT TCT CCC CTA GTG AGA GAA AGA GGT CCT CAG AGA GTA GCA GCT 912

305 H I T G T R G R S N T L S S P N 320
913 CAC ATA ACT GGG ACC AGA GGA AGA AGC AAC ACA TTG TCT TCT CCA AAC 960

321 S K N E K A L G R K I N S W E S 336
961 TCC AAG AAT GAA AAG GCT CTG GGC CGC AAA ATA AAC TCC TGG GAA TCA 1008

337 S R S G H S F L S N L H L R N G 352
1009 TCA AGG AGT GGG CAT TCA TTC CTG AGC AAC TTG CAC TTG AGG AAT GGT 1056

353 E L V I H E K G F Y Y I Y S Q T 368
1057 GAA CTG GTC ATC CAT GAA AAA GGG TTT TAC TAC ATC TAT TCC CAA ACA 1104

369 Y F R F Q E E I K E N T K N D K 384
1105 TAC TTT CGA TTT CAG GAG GAA ATA AAA GAA AAC ACA AAG AAC GAC AAA 1152

385 Q M V Q Y I Y K Y T S Y P D P I 400
1153 CAA ATG GTC CAA TAT ATT TAC AAA TAC ACA AGT TAT CCT GAC CCT ATA 1200

401 L L M K S A R N S C W S K D A E 416
1201 TTG TTG ATG AAA AGT GCT AGA AAT AGT TGT TGG TCT AAA GAT GCA GAA 1248

417 Y G L Y S I Y Q G G I F E L K E 432
1249 TAT GGA CTC TAT TCC ATC TAT CAA GGG GGA ATA TTT GAG CTT AAG GAA 1296

433 N D R I F V S V T N E H L I D M 448
1297 AAT GAC AGA ATT TTT GTT TCT GTA ACA AAT GAG CAC TTG ATA GAC ATG 1344

449 D H E A S F F G A F L V G * 462
1345 GAC CAT GAA GCC AGT TTT TTC GGG GCC TTT TTA GTT GGC TAA 1386
**Merkmale Konstrukt C:**
Leitpeptidsequenz: NT 1-57, AA 1-19
Sequenz des Einzelketten (scFv)-Antikörperfragmentes 40 spezifisch für das Tumorstroma-Antigen FAP: NT 58-801, AA 20-267
Sequenz des Linkers zwischen scFv und TRAIL-Fragment (AA 95-281): NT 802-822, AA 268-274
Sequenz des humanen TRAIL Fragmentes (extrazelluläre Domäne, AA 95-281 des natürlichen, humanen TRAIL Moleküls): NT 823-1383 AA 275-461
Stop Kodon: NT 1384-1386.
**Sequenzprotokoll zu Patentanmeldung AMAIZe**
   **AZ#**
   **Anmeldernummer:**
**Sequenzbeschreibung 4:** Kodierende DNA Sequenz (unten, nur kodierender DNA Strang, Nukleotid (NT) 1-1254)und translatierte Aminosäuresequenz (oben, Einzelbuchstaben Kodierung der Aminosäuren (AA) 1-417)eines erfindungsgemäßen Antikörper-Zytokin-Fusionsproteins AMAIZe am Beispiel von FasL-AMAIZe(40)(Konstrukt D). 
1 M D W T W R V F C L L A V A P G 16
1 ATG GAC TGG ACC TGG CGC GTG TTT TGC CTG CTC GCC GTG GCT CCT GGG 48

17 A H S Q V Q L V Q S G G G M V E 32
49 GCC CAC AGC CAG GTA CAG CTG GTG CAG TCT GGG GGA GGC ATG GTA GAG 96

33 P G G S L R L S C A A S G F T F 48
97 CCT GGG GGG TCC CTT AGA CTC TCC TGT GCA GCC TCT GGA TTC ACT TTC 144

49 S N A W M S W V R Q A P G K G L 64
145 AGT AAT GCC TGG ATG AGC TGG GTC CGC CAG GCT CCA GGG AAG GGG CTG 192

65 E W V G R I K S K A G G G T A E 80
193 GAG TGG GTT GGC CGT ATA AAA AGC AAA GCT GGT GGT GGG ACA GCA GAG 240

81 Y A A P V K G R F T I S R D D S 96
241 TAC GCT GCA CCC GTG AAA GGC AGA TTC ACC ATC TCA AGA GAT GAT TCA 288

97 Q N T L Y L Q M N S L K T D D T 112
289 CAA AAC ACG CTG TAT CTG CAA ATG AAC AGC CTG AAA ACC GAC GAC ACA 336

113 A V Y Y C T T H V Y G A P R N W 128
337 GCC GTG TAT TAC TGT ACC ACA CAT GTC TAC GGT GCC CCC CGG AAC TGG 384

129 G Q G S L V T V S S A S T K G P 144
385 GGC CAG GGA TCC CTG GTC ACC GTC TCC TCA GCC TCC ACC AAG GGC CCA 432

145 K L E E G E F S E A R V Q S V L 160
433 AAG CTT GAA GAA GGT GAA TTT TCA GAA GCA CGC GTA CAG TCT GTG TTG 480

161 T Q P P S V S A A P G Q K V T I 176
481 ACT CAG CCG CCC TCA GTG TCT GCG GCC CCA GGA CAG AAG GTC ACC ATC 528

177 S C S G S S S N I G N N Y V S W 192
529 TCC TGC TCT GGA AGC AGC TCC AAC ATT GGA AAT AAT TAT GTC TCC TGG 576

193 Y V Q L P G T A P K L L I Y D N 208
577 TAC GTT CAA CTC CCA GGA ACA GCC CCC AAA CTC CTC ATT TAT GAC AAT 624

209 N K R F S G V P D R F S G S K S 224
625 AAT AAG CGA TTC TCA GGA GTT CCT GAC CGA TTC TCT GGC TCC AAG TCT 672

225 G T S A T L G I T G L Q T G D E 240
673 GGC ACG TCA GCC ACC CTG GGC ATC ACC GGG CTC CAG ACT GGG GAC GAG 720

241 A D Y Y C G A W D G S L R E A V 256
721 GCC GAT TAT TAC TGC GGA GCA TGG GAT GGC AGC CTG CGT GAA GCG GTA 768

257 F G G G T K V T V L G A A A V E 272
769 TTC GGC GGA GGG ACC AAG GTC ACC GTC CTA GGT GCG GCC GCA GTT GAG 816
 
273 L E K K E L R K V A H L T G K 288
817 CTC GAG GAA AAA AAG GAG CTG AGG AAA GTG GCC CAT TTA ACA GGC AAG 864

289 S N S R S M P L E W E D T Y G I 304
865 TCC AAC TCA AGG TCC ATG CCT CTG GAA TGG GAA GAC ACC TAT GGA ATT 912

305 V L L S G V K Y K K G G L V I N 320
913 GTC CTG CTT TCT GGA GTG AAG TAT AAG AAG GGT GGC CTT GTG ATC AAT 960

321 E T G L Y F V Y S K V Y F R G Q 336
961 GAA ACT GGG CTG TAC TTT GTA TAT TCC AAA GTA TAC TTC CGG GGT CAA 1008

337 S C N N L P L S H K V Y M R N S 352
1009 TCT TGC AAC AAC CTG CCC CTG AGC CAC AAG GTC TAC ATG AGG AAC TCT 1056

353 K Y P Q D L V M M E G K M M S Y 368
1057 AAG TAT CCC CAG GAT CTG GTG ATG ATG GAG GGG AAG ATG ATG AGC TAC 1104

369 C T T G Q M W A R S S Y L G A V 384
1105 TGC ACT ACT GGG CAG ATG TGG GCC CGC AGC AGC TAC CTG GGG GCA GTG 1152

385 F N L T S A D H L Y V N V S E L 400
1153 TTC AAT CTT ACC AGT GCT GAT CAT TTA TAT GTC AAC GTA TCT GAG CTC 1200

401 S L V N F E E S Q T F F G L Y K 416
1201 TCT CTG GTC AAT TTT GAG GAA TCT CAG ACG TTT TTC GGC TTA TAT AAG 1248

417 L * 418
1249 CTC TAA 1254
**Merkmale Konstrukt D:**
Leitpeptidsequenz: NT 1-57, AA 1-19
Sequenz des Einzelketten (scFv)-Antikörperfragmentes 40 spezifisch für das Tumorstroma-Antigen FAP: NT 58-801, AA 20-267
Sequenz des Linkers zwischen scFv und FasL-Fragment (AA 139-281): NT 802-822, AA 268-274
Sequenz des humanen FasL Fragmentes (extrazelluläre Domäne, AA 139-281 des natürlichen, humanen FasL Moleküls): NT 823-1251, AA 275-417
Stop Kodon: NT 1552-1554.
**Sequenzprotokoll zu Patentanmeldung AMAIZe**
   **AZ#**
   **Anmeldernummer:**
**Sequenzbeschreibung 5:** Kodierende DNA Sequenz (unten, nur kodierender DNA Strang, Nukleotid (NT) 1-1299)und translatierte Aminosäuresequenz (oben, Einzelbuchstaben Kodierung der Aminosäuren (AA) 1-432)eines erfindungsgemäßen Antikörper-Zytokin-Fusionsproteins AMAIZe am Beispiel von FasL-AMAIZe(OS4)(Konstrukt E). 
1 M D W T W R V F C L L A V A P G 16
1 ATG GAC TGG ACC TGG CGC GTG TTT TGC CTG CTC GCC GTG GCT CCT GGG 48

17 A H S Q V Q L V Q S G A E V K K 32
49 GCC CAC AGC CAG GTG CAA CTA GTG CAG TCC GGC GCC GAA GTG AAG AAA 96

33 P G A S V K V S C K T S R Y T F 48
97 CCC GGT GCT TCC GTG AAA GTC AGC TGT AAA ACT AGT AGA TAC ACC TTC 144

49 T E Y T I H W V R Q A P G Q R L 64
145 ACT GAA TAC ACC ATA CAC TGG GTT AGA CAG GCC CCT GGC CAA AGG CTG 192

65 E W I G G I N P N N G I P N Y N 80
193 GAG TGG ATA GGA GGT ATT AAT CCT AAC AAT GGT ATT CCT AAC TAC AAC 240

81 Q K F K G R V T I T V D T S A S 96
241 CAG AAG TTC AAG GGC CGG GTC ACC ATC ACC GTA GAC ACC TCT GCC AGC 288

97 T A Y M E L S S L R S E D T A V 112
289 ACC GCC TAC ATG GAA CTG TCC AGC CTG CGC TCC GAG GAC ACT GCA GTC 336

113 Y Y C A R R R I A Y G Y D E G H 128
337 TAC TAC TGC GCC AGA AGA AGA ATC GCC TAT GGT TAC GAC GAG GGC CAT 384

129 A M D Y W G Q G T L V T V S S A 144
385 GCT ATG GAC TAC TGG GGT CAA GGA ACC CTT GTC ACC GTC TCC TCA GCC 432

145 S T K G P K L E E G E F S E A R 160
433 TCC ACC AAG GGC CCA AAG CTT GAA GAA GGT GAA TTT TCA GAA GCA CGC 480

161 V D I V M T Q S P D S L A V S L 176
481 GTA GAC ATT GTG ATG ACC CAA TCT CCA GAC TCT TTG GCT GTG TCT CTA 528

177 G E R A T I N C K S S Q S L L Y 192
529 GGG GAG AGG GCC ACC ATC AAC TGC AAG TCC AGT CAG AGC CTT TTA TAT 576

193 S R N Q K N Y L A W Y Q Q K P G 208
577 TCT AGA AAT CAA AAG AAC TAC TTG GCC TGG TAT CAG CAG AAA CCA GGA 624

209 Q P P K L L I F W A S T R E S G 224
625 CAG CCA CCC AAA CTC CTC ATC TTT TGG GCT AGC ACT AGG GAA TCT GGG 672

225 V P D R F S G S G F G T D F T L 240
673 GTA CCT GAT AGG TTC AGT GGC AGT GGG TTT GGG ACA GAC TTC ACC CTC 720

241 T I S S L Q A E D V A V Y Y C Q 256
721 ACC ATT AGC AGC CTG CAG GCT GAA GAT GTG GCA GTT TAT TAC TGT CAG 768

257 Q Y F S Y P L T F G Q G T K V E 272
769 CAA TAT TTT AGC TAT CCG CTC ACG TTC GGA CAA GGG ACC AAG GTG GAA 816
 
273 I K R T V A A P S V F A V F A A 288
817 ATA AAA CGT ACT GTG GCT GCA CCA TCT GTC TTC GCT GTC TTC GCG GCC 864

289 A E K K E L R K V A H L T G K S 304
865 GCA GAA AAA AAG GAG CTG AGG AAA GTG GCC CAT TTA ACA GGC AAG TCC 912

305 N S R S M P L E W E D T Y G I V 320
913 AAC TCA AGG TCC ATG CCT CTG GAA TGG GAA GAC ACC TAT GGA ATT GTC 960

321 L L S G V K Y K K G G L V I N E 336
961 CTG CTT TCT GGA GTG AAG TAT AAG AAG GGT GGC CTT GTG ATC AAT GAA 1008

337 T G L Y F V Y S K V Y F R G Q S 352
1009 ACT GGG CTG TAC TTT GTA TAT TCC AAA GTA TAC TTC CGG GGT CAA TCT 1056

353 C N N L P L S H K V Y M R N S K 368
1057 TGC AAC AAC CTG CCC CTG AGC CAC AAG GTC TAC ATG AGG AAC TCT AAG 1104

369 Y P Q D L V M M E G K M M S Y C 384
1105 TAT CCC CAG GAT CTG GTG ATG ATG GAG GGG AAG ATG ATG AGC TAC TGC 1152

385 T T G Q M W A R S S Y L G A V F 400
1153 ACT ACT GGG CAG ATG TGG GCC CGC AGC AGC TAC CTG GGG GCA GTG TTC 1200

401 N L T S A D H L Y V N V S E L S 416
1201 AAT CTT ACC AGT GCT GAT CAT TTA TAT GTC AAC GTA TCT GAG CTC TCT 1248

417 L V N F E E S Q T F F G L Y K L 432
1249 CTG GTC AAT TTT GAG GAA TCT CAG ACG TTT TTC GGC TTA TAT AAG CTC 1296

433 * 433
1297 TAA 1299
**Merkmale:**
Leitpeptidsequenz: NT 1-57, AA 1-19
Sequenz des Einzelketten (scFv)-Antikörperfragmentes OS4 spezifisch für das Tumorstroma-Antigen FAP: NT 58-822, AA 20-274
Sequenz des Linkers zwischen scFv und FasL-Fragment (AA 139-281): NT 823-867, AA 275-289
Sequenz des humanen FasL Fragmentes (extrazelluläre Domäne, AA 139-281 des natürlichen, humanen FasL Moleküls): NT 868-1296 AA 290-432
Stop Kodon: NT 1441-1443.
**Sequenzprotokoll zu Patentanmeldung AMAIZe**
   **AZ#**
   **Anmeldernummer:**
**Sequenzbeschreibung 6:** Kodierende DNA Sequenz (unten, nur kodierender DNA Strang, Nukleotid (NT) 1-1278)und translatierte Aminosäuresequenz (oben, Einzelbuchstaben Kodierung der Aminosäuren (AA) 1-425)eines erfindungsgemäßen Antikörper-Zytokin-Fusionsproteins AMAIZe am Beispiel von FasL-AMAIZe(40-Flag)(Konstrukt F). 
1 M D W T W R V F C L L A V A P G 16
1 ATG GAC TGG ACC TGG CGC GTG TTT TGC CTG CTC GCC GTG GCT CCT GGG 48

17 A H S Q V Q L V Q S G G G M V E 32
49 GCC CAC AGC CAG GTA CAG CTG GTG CAG TCT GGG GGA GGC ATG GTA GAG 96

33 P G G S L R L S C A A S G F T F 48
97 CCT GGG GGG TCC CTT AGA CTC TCC TGT GCA GCC TCT GGA TTC ACT TTC 144

49 S N A W M S W V R Q A P G K G L 64
145 AGT AAT GCC TGG ATG AGC TGG GTC CGC CAG GCT CCA GGG AAG GGG CTG 192

65 E W V G R I K S K A G G G T A E 80
193 GAG TGG GTT GGC CGT ATA AAA AGC AAA GCT GGT GGT GGG ACA GCA GAG 240

81 Y A A P V K G R F T I S R D D S 96
241 TAC GCT GCA CCC GTG AAA GGC AGA TTC ACC ATC TCA AGA GAT GAT TCA 288

97 Q N T L Y L Q M N S L K T D D T 112
289 CAA AAC ACG CTG TAT CTG CAA ATG AAC AGC CTG AAA ACC GAC GAC ACA 336

113 A V Y Y C T T H V Y G A P R N W 128
337 GCC GTG TAT TAC TGT ACC ACA CAT GTC TAC GGT GCC CCC CGG AAC TGG 384

129 G Q G S L V T V S S A S T K G P 144
385 GGC CAG GGA TCC CTG GTC ACC GTC TCC TCA GCC TCC ACC AAG GGC CCA 432

145 K L E E G E F S E A R V Q S V L 160
433 AAG CTT GAA GAA GGT GAA TTT TCA GAA GCA CGC GTA CAG TCT GTG TTG 480

161 T Q P P S V S A A P G Q K V T I 176
481 ACT CAG CCG CCC TCA GTG TCT GCG GCC CCA GGA CAG AAG GTC ACC ATC 528

177 S C S G S S S N I G N N Y V S W 192
529 TCC TGC TCT GGA AGC AGC TCC AAC ATT GGA AAT AAT TAT GTC TCC TGG 576

193 Y V Q L P G T A P K L L I Y D N 208
577 TAC GTT CAA CTC CCA GGA ACA GCC CCC AAA CTC CTC ATT TAT GAC AAT 624

209 N K R F S G V P D R F S G S K S 224
625 AAT AAG CGA TTC TCA GGA GTT CCT GAC CGA TTC TCT GGC TCC AAG TCT 672

225 G T S A T L G I T G L Q T G D E 240
673 GGC ACG TCA GCC ACC CTG GGC ATC ACC GGG CTC CAG ACT GGG GAC GAG 720

241 A D Y Y C G A W D G S L R E A V 256
721 GCC GAT TAT TAC TGC GGA GCA TGG GAT GGC AGC CTG CGT GAA GCG GTA 768

257 F G G G T K V T V L G D Y K D D 272
769 TTC GGC GGA GGG ACC AAG GTC ACC GTC CTA GGT GAT TAC AAA GAC GAT 816
 
273 D D K A A A V E L E E K K E L R 288
817 GAC GAT AAA GCG GCC GCA GTT GAG CTC GAG GAA AAA AAG GAG CTG AGG 864

289 K V A H L T G K S N S R S M P L 304
865 AAA GTG GCC CAT TTA ACA GGC AAG TCC AAC TCA AGG TCC ATG CCT CTG 912

305 E W E D T Y G I V L L S G V K Y 320
913 GAA TGG GAA GAC ACC TAT GGA ATT GTC CTG CTT TCT GGA GTG AAG TAT 960

321 K K G G L V I N E T G L Y F V Y 336
961 AAG AAG GGT GGC CTT GTG ATC AAT GAA ACT GGG CTG TAC TTT GTA TAT 1008

337 S K V Y F R G Q S C N N L P L S 352
1009 TCC AAA GTA TAC TTC CGG GGT CAA TCT TGC AAC AAC CTG CCC CTG AGC 1056

353 H K V Y M R N S K Y P Q D L V M 368
1057 CAC AAG GTC TAC ATG AGG AAC TCT AAG TAT CCC CAG GAT CTG GTG ATG 1104

369 M E G K M M S Y C T T G Q M W A 384
1105 ATG GAG GGG AAG ATG ATG AGC TAC TGC ACT ACT GGG CAG ATG TGG GCC 1152

385 R S S Y L G A V F N L T S A D H 400
1153 CGC AGC AGC TAC CTG GGG GCA GTG TTC AAT CTT ACC AGT GCT GAT CAT 1200

401 L Y V N V S E L S L V N F E E S 416
1201 TTA TAT GTC AAC GTA TCT GAG CTC TCT CTG GTC AAT TTT GAG GAA TCT 1248

417 Q T F F G L Y K L * 426
1249 CAG ACG TTT TTC GGC TTA TAT AAG CTC TAA 1278
**Merkmale Konstrukt F:**
Leitpeptidsequenz: NT 1-57, AA 1-19
Sequenz des Einzelketten (scFv)-Antikörperfragmentes 40 spezifisch für das Tumorstroma-Antigen FAP: NT 58-801, AA 20-267
Sequenz des Flag-tag zwischen scFv und Linker-Sequenz: NT 802-825, AA 268-275
Sequenz von des Linkers zwischen Flag-tag und FasL-Fragment (AA 139-281): NT 826-846, AA 276-282
Sequenz des humanen FasL Fragmentes (extrazelluläre Domäne, AA 139-281 des natürlichen, humanen FasL Moleküls): NT 847-1275, AA 283-425
Stop Kodon: NT 1276-1278.

## Patentansprüche

1. Polypeptid mit proapoptotischen und immunmodulierenden Eigenschaften, **dadurch gekennzeichnet, dass** es (i) einen Abschnitt (1), bestehend aus der Aminosäuresequenz einer extrazellulären Domäne eines Mitglieds der TNF-Familie, (ii) N-terminal von Abschnitt (1) einen Abschnitt (2), welcher ein Peptidlinker ist, und (iii) einen Abschnitt (3), welcher ein an ein zellmembranständiges Antigen bindendes Antikörperfragment ist, enthält, wobei das Polypeptid einen zellmembranständigen Zytokinrezeptor erkennt, und wobei das Antikörperfragment als scFv vorliegt.

2. Polypeptid nach Anspruch 1, **dadurch gekennzeichnet, dass** der Abschnitt (1) eine Aminosäuresequenz einer extrazellulären Domäne von TRAIL (TNF Related Apoptosis Inducing Ligand, AA 95-281, NCBI Accession No AAC50332) oder von FasL (AA 139-281, NCBI Accession No. AAC50124) enthält.

3. Polypeptid nach einem der vorgenannten Ansprüche, **dadurch gekennzeichnet, dass** der Abschnitt (3) ein Antikörperfragment eines Säugetiers, insbesondere murinen, oder humanen Ursprungs, ist oder ein humanisiertes Antikörperfragment ist.

4. Nukleinsäurekonstrukt, **dadurch gekennzeichnet, dass** es eine Nukleotidsequenz, codierend für ein Polypeptid nach einem der vorgenannten Ansprüche, enthält.

5. Vektor, **dadurch gekennzeichnet, dass** er ein Nukleinsäurekonstrukt gemäß Anspruch 4 enthält.

6. Wirtszelle, **dadurch gekennzeichnet, dass** sie eine Nukleinsäure gemäß Anspruch 4 und/oder einen Vektor gemäß Anspruch 5 enthält.

7. Verfahren zur Isolierung eines Polypeptids nach einem der Ansprüche 1 bis 3, **dadurch gekennzeichnet, dass**
(a) ein Vektor gemäß Anspruch 5 oder ein Nukleinsäurekonstrukt gemäß Anspruch 4 hergestellt wird,
(b) Zellen mit einem gemäß Verfahrensschritt (a) erhaltenen Vektor oder Nukleinsäurekonstrukt transfiziert werden,
(c) die gemäß (b) transfizierten Zellen kultiviert werden, und
(d) unter entsprechenden Bedingungen exprimierte Polypeptide aus den Wirtszellen und/oder dem Kulturüberstand isoliert werden.

8. Verwendung eines Polypeptids nach einem der Ansprüche 1 bis 3, eines Nukleinsäurekonstrukts nach Anspruch 4, eines Vektors nach Anspruch 5 oder einer Wirtszelle nach Anspruch 6 zur Herstellung eines Arzneimittels.

9. Verwendung eines Polypeptids nach einem der Ansprüche 1 bis 3, eines Nukleinsäurekonstrukts nach Anspruch 4, eines Vektors nach Anspruch 5 oder einer Wirtszelle nach Anspruch 6 zur Herstellung eines Medikaments zur Behandlung von Krebserkrankungen, insbesondere soliden oder lymphatischen Tumoren, Infektionskrankheiten, Stoffwechselerkrankungen, Entzündungszuständen, Autoimmunerkrankungen, insbesondere rheumato/arthritische Erkrankungen.

10. Zusammensetzung, enthaltend Polypeptide einem der Ansprüche 1 bis 3, Nukleinsäurekonstrukte nach Anspruch 4, Vektoren nach Anspruch 5 und/oder Wirtszellen nach Anspruch 6 sowie pharmazeutisch unbedenkliche Hilfs-, Zusatz- und/oder Trägersubstanzen.

11. Verwendung einer Zusammensetzung nach Anspruch 10 zur Herstellung eines Arzneimittels, insbesondere zur Behandlung von Krebserkrankungen, insbesondere soliden oder lymphatischen Tumoren, Infektionskrankheiten, Stoffwechselerkrankungen, Entzündungszuständen, Autoimmunerkrankungen, insbesondere rheumato/arthritische Erkrankungen.

## Claims

1. A polypeptide having proapoptotic and immunomodulating properties, comprising: (i) a segment (1) consisting of the amino acid sequence of an extracellular domain of a member of the TNF family; (ii) N-terminally of segment (1) a segment (2) which is a peptide linker; and (iii) a segment (3) which is an antibody fragment that binds to an antigen on the cell membrane, wherein the polypeptide recognizes a cytokine receptor on the cell membrane and wherein the antibody fragment is present as scFv.

2. The polypeptide of claim 1, wherein the segment (1) comprises an amino acid sequence of an extracellular domain of TRAIL (TNF Related Apoptosis Inducing Ligand, AA 95-281, NCBI Accession No. AAC50332) or of FasL (AA 139-281, NCBI Accession No. AAC50124).

3. The polypeptide of any one of the preceding claims, wherein segment (3) is an antibody fragment of a mammal, in particular of murine or human origin, or a humanised antibody fragment.

4. A nucleic acid construct comprising a nucleotide sequence encoding a polypeptide of any one of the preceding claims.

5. A vector comprising a nucleic acid construct of claim 4.

6. A host cell comprising a nucleic acid of claim 4 and/or a vector of claim 5.

7. A method for the isolation of a polypeptide of any one of claims 1 to 3 comprising:
(a) producing a vector of claim 5 or a nucleic acid construct of claim 4;
(b) transfecting cells with the vector or nucleic acid construct obtained according to step (a);
(c) culturing the cells transfected according to step (b); and
(d) isolating polypeptides expressed under appropriate conditions from the host cells and/or the culture supernatant.

8. Use of a polypeptide of any one of claims 1 to 3, a nucleic acid construct of claim 4, a vector of claim 5 or a host cell of claim 6 for preparing a medicament.

9. Use of a polypeptide of any one of claims 1 to 3, a nucleic acid construct of claim 4, a vector of claim 5 or a host cell of claim 6 for preparing a medicament for treating cancer diseases, in particular solid or lymphatic tumors, infectious diseases, metabolic diseases, inflammatory conditions, autoimmune diseases, in particular rheumatoid arthritic diseases.

10. A composition comprising polypeptides of any one of claims 1 to 3, nucleic acid constructs of claim 4, vectors of claim 5 and/or host cells of claim 6 and pharmaceutically acceptable excipients, additives, and/or carriers.

11. Use of a composition of claim 10 for preparing a medicament, in particular for treating cancer diseases, in particular solid or lymphatic tumors, infectious diseases, metabolic diseases, inflammatory conditions, autoimmune diseases, in particular rheumatoid arthritic diseases.

## Revendications

1. Polypeptide ayant des propriétés pro-apoptotiques et immunomodulatrices, **caractérisé en ce qu'**il contient (i) une portion (1), consistant en la séquence d'acides aminés d'un domaine extracellulaire d'un membre de la famille du TNF, (ii) à l'extrémité N-terminale de la portion (1) une portion (2), qui est un lieur peptidique, et (iii) une portion (3) qui est un fragment d'anticorps de liaison à un antigène présent dans la membrane cellulaire, tandis que le polypeptide reconnaît un récepteur de cytokine présent dans la membrane cellulaire, et tandis que le fragment d'anticorps agit comme scFv.

2. Polypeptide selon la revendication 1, **caractérisé en ce que** la portion (1) contient une séquence d'acides aminés d'un domaine extracellulaire de TRAIL (TNF Related Apoptosis Inducing Ligand, AA 95-281, NCBI No. d'accès AAC50332) ou de FasL (AA 139-281, NCBI No. d'accès AAC50124).

3. Polypeptide selon l'une des revendications précédentes, **caractérisé en ce que** la portion (3) est un fragment d'anticorps d'un mammifère, en particulier d'origine murine ou humaine, ou est un fragment d'anticorps humanisé.

4. Construction d'acide nucléique, **caractérisée en ce qu'**elle contient une séquence nucléotidique codant pour un polypeptide selon l'une des revendications précédentes.

5. Vecteur, **caractérisé en ce qu'**il contient une construction d'acide nucléique selon la revendication 4.

6. Cellule hôte, **caractérisée en ce qu'**elle contient un acide nucléique selon la revendication 4 et/ ou un vecteur selon la revendication 5.

7. Procédé pour l'isolement d'un polypeptide selon l'une des revendications 1 à 3, **caractérisé en ce que**
(a) on prépare un vecteur selon la revendication 5 ou une construction d'acide nucléique selon le revendication 4,
(b) on transfecte des cellules avec un vecteur ou une construction d'acide nucléique obtenu selon l'étape opératoire (a),
(c) on cultive les cellules transfectées selon (b), et
(d) on isole dans des conditions appropriées les polypeptides exprimés à partir des cellules hôtes et/ou du surnageant de culture.

8. Utilisation d'un polypeptide selon l'une des revendications 1 à 3, d'une construction d'acide nucléique selon la revendication 4, d'un vecteur selon la revendication 5 ou d'une cellule hôte selon la revendication 6 pour la préparation d'un médicament.

9. Utilisation d'un polypeptide selon l'une des revendications 1 à 3, d'une construction d'acide nucléique selon la revendication 4, d'un vecteur selon la revendication 5 ou d'une cellule hôte selon la revendication 6 pour la préparation d'un médicament pour le traitement d'affections cancéreuses, en particulier de tumeurs solides ou lymphatiques, de maladies infectieuses, de troubles du métabolisme, d'états inflammatoires, de maladies auto-immunes, en particulier d'affections rhumato/arthritiques.

10. Composition, contenant des polypeptides selon l'une des revendications 1 à 3, des constructions d'acide nucléique selon la revendication 4, des vecteurs selon la revendication 5 et/ou des cellules hôtes selon la revendication 6, ainsi que des substances auxiliaires, additionnelles et/ou de support pharmaceutiquement acceptables.

11. Utilisation d'une composition selon la revendication 10 pour la préparation d'un médicament, en particulier pour le traitement d'affections cancéreuses, en particulier de tumeurs solides ou lymphatiques, de maladies infectieuses, d'affections du métabolisme, d'états inflammatoires, d'affections auto-immunes, en particulier d'affections rhumato/arthritiques.
